# EUROPEAN PATENT APPLICATION

(11) **EP 4 406 935 A1**
(43) Date of publication of application: **31.07.2024**
(21) Application number: 22872031.4
(22) Date of filing: 21.09.2022
(51) Int. Cl.: C07C 57/13, C07C 57/42, C07C 61/35, C07C 55/28, A61P 3/10, A61P 3/04, A61P 35/00, A61K 31/194

(54) **LONG-CHAIN COMPOUND THAT ACTS ON ACLY, PREPARATION METHOD THEREFOR AND USE THEREOF**

(30) Priority: 23.09.2021 CN 202111116425
(71) Applicant: Shanghai Institute of Materia Medica, Chinese Academy of Sciences, Pudong, Shanghai 201203 (CN)
(72) Inventor: NAN, Fajun, Shanghai 201203 (CN); LI, Jingya, Shanghai 201203 (CN); CHEN, Yi, Shanghai 201203 (CN); SONG, Gaolei, Shanghai 201203 (CN); XIE, Zhifu, Shanghai 201203 (CN); FANG, Yanfen, Shanghai 201203 (CN); ZHANG, Mei, Shanghai 201203 (CN); SUN, Xinyu, Shanghai 201203 (CN); CAO, Lei, Shanghai 201203 (CN); MA, Hui, Shanghai 201203 (CN); YANG, Yurou, Shanghai 201203 (CN)
(74) Representative: Lavoix
(86) International application number: PCT/CN2022/120328
(87) International publication number: WO 2023/045986

(57) **Abstract**

A long-chain compound, a preparation method therefor and the use thereof are disclosed. The structure of said compound is represented by formula (I). The definition of each substituent in the formula is as set out in the description and claims. The compound of the present invention can directly inhibit ACLY, inhibit lipid synthesis in primary hepatocytes, inhibit lipid de novo synthesis and histone acetylation in various cancer cells such as H358, and inhibit cancer cell proliferation. The compound may be used to prepare drugs that treat metabolic diseases such as hyperlipidemia and atherosclerosis or various cancers such as lung cancer, pancreatic cancer, breast cancer, ovarian cancer, liver cancer, intestinal cancer, brain cancer, and acute myeloid leukemia.

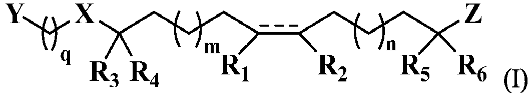

## Description

### Technical field

The present invention belongs to the field of medicinal chemistry and relates to a new type of ACLY inhibitors or pharmaceutically acceptable salts thereof and preparation method therefor, as well as a pharmaceutical combination containing such inhibitors. This type of inhibitor can directly inhibit ACLY, inhibit lipid synthesis in primary hepatocytes, inhibit de novo lipid synthesis, histone acetylation, and inhibit proliferation in various cancer cells such as H358, and can be used to prepare a medicament for metabolic diseases such as hyperlipidemia, atherosclerosis, non-alcoholic steatohepatitis, or for various cancer diseases such as lung cancer, pancreatic cancer, breast cancer, ovarian cancer, liver cancer, intestinal cancer, brain cancer, acute myeloid leukemia and the like.

### Background technology

ATP citrate lyase (ACLY) is at a key position in glucose and lipid metabolism. Acetyl coenzyme A (Ac-CoA) produced in mitochondria cannot directly pass through the mitochondrial membrane to reach the cytoplasm, but AC-CoA can enter the tricarboxylic acid cycle. Citric acid produced by the tricarboxylic acid cycle is transported to the cytoplasm via the citrate transporter protein in the mitochondrial membrane. In the cytoplasm, citrate and coenzyme A (CoA) can be catalyzed by ACLY to produce acetyl coenzyme A and oxaloacetate while consuming one molecule of ATP. The biological functions of acetyl coenzyme A in the body can be summarized in three ways: in the fatty acid synthesis pathway, it can be carboxylated by acetyl coenzyme A carboxylase (ACC) to form malonyl coenzyme A, which is then subjected to the relevant fatty acid synthases to ultimately produce fatty acids; acetyl coenzyme A is likewise a precursor of the mevalonate pathway, which synthesizes farnesyl pyrophosphate (FPP), which is involved in cholesterol synthesis; in addition, acetyl coenzyme A also provides a raw material for acetylation reactions and is involved in the acetylation of a variety of proteins, including histones. ACLY is involved in lipid synthesis and epigenetic regulation in vivo by regulating acetyl coenzyme A in vivo.

Various metabolic diseases such as hyperlipidemia, atherosclerosis, and non-alcoholic fatty liver disease are related to the increased level of auto-lipid synthesis. ACLY, as the main enzyme that generates Ac-CoA in the cytoplasm, provides a raw material for lipid synthesis, and is the key link in lipid synthesis, and Bempedoic acid, a drug that acts on ACLY, significantly reduces the level of lipid synthesis in the liver. In addition, the lipid-lowering effects of ACLY inhibitors such as benzenesulfonamides have been verified at the cellular level.

Metabolic reorganization is the most common and dominant feature of cancer cells. This is due to the fact that the rapid growth of cancer cells requires large amounts of energy and macromolecules, and in order to satisfy this "demand", their metabolism will undergo significant changes. Since cancerous tissues often develop into irregularly shaped masses, it becomes extremely difficult for cancer cells to obtain lipids from the surrounding blood vessels, and de novo lipid synthesis of their own lipids is usually at a high level, which is the main source of lipids for their proliferation. Abnormal epigenetic regulation is another major feature of cancer cells, as a key link of epigenetic regulation, histone acetylation has a very important role, when the level of histone acetylation is elevated, the chromatin structure is relaxed, which is in the state of transcriptional activation; on the contrary, when the level of histone acetylation is low, the chromatin structure is tight, which is in the state of transcriptional repression. Studies have shown that the occurrence of many cancers is closely related to elevated levels of histone acetylation. Acetyl coenzyme A catalyzed by ACLY is a raw material for lipid synthesis and also involved in histone acetylation. Studies have shown that ACLY is highly expressed in a variety of cancers, including non-small cell lung cancer, breast cancer, and liver cancer, and the high expression of ACLY is closely related to the poor prognosis of related cancers.

In summary, the development of ACLY inhibitors is expected to be used in the clinical treatment of metabolic diseases and cancers.

### Summary of the invention

An object of the present invention is to provide a novel class of compounds having ACLY inhibitory activity or pharmaceutically acceptable salts thereof, including, but not limited to, sodium, potassium, magnesium, calcium salts formed from the carboxylic acid moiety of said compounds, or inorganic acid salts formed when the compounds contain nitrogen.

The first aspect of the present invention provides a compound of the general formula (I), or a stereoisomer, enantiomer, diastereoisomer, racemate, or a pharmaceutically acceptable salt thereof, 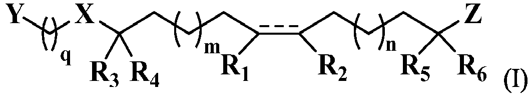
wherein --- represents a double bond or a single bond;
R₁ and R₂ are each independently H, C1-C4 alkyl, C2-C4 alkenyl, C2-C4 alkynyl, C3-C7 cycloalkyl, C3-C7 cycloalkenyl or C6-C10 aryl; or, R₁ and R₂ together with the attached carbons form a saturated 3-7 membered ring, or a 3-7 membered ring containing an unsaturated double bond;
R₃ and R₄ are each independently H, C1-C4 alkyl, C2-C4 alkenyl, C2-C4 alkynyl, C3-C7 cycloalkyl, C3-C7 cycloalkenyl or C6-C10 aryl; or, R₃ and R₄ together with the attached carbon form a saturated 3-7 membered ring, or a 3-7 membered ring containing an unsaturated double bond;
R₅ and R₆ are each independently H, C1-C4 alkyl, C2-C4 alkenyl, C2-C4 alkynyl, C3-C7 cycloalkyl, C3-C7 cycloalkenyl or C6-C10 aryl; or, R₅ and R₆ together with the attached carbon form a saturated 3-7 membered ring, or a 3-7 membered ring containing an unsaturated double bond;
m, n and q are each independently 0, 1, 2, 3, 4, 5 or 6;
Z is -OH, -COOH, -COOR₇, -SO₃H or -CONHR₇, wherein R₇ is C1-C4 alkyl;
X is -CO-, -O-, -NH-, -S-, -CH₂-, 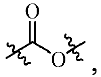 -CONH-, 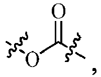 -NHOC-, 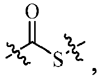 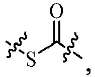 -CH₂CO- or -COCH₂-;
Y is H, C6-C10 aryl, C1-C4 alkyl or adamantyl (Ad-); or Y is: 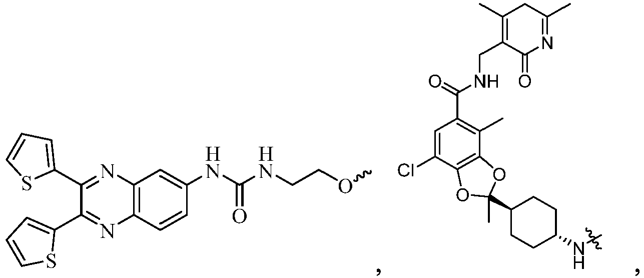 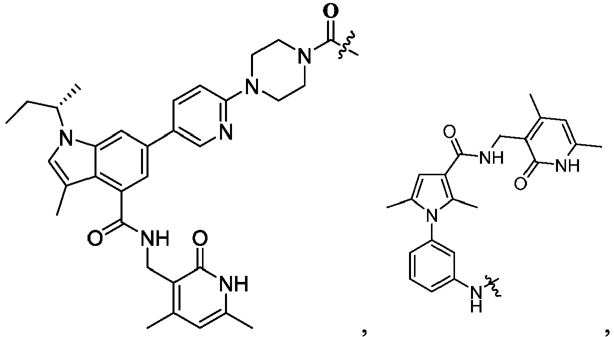 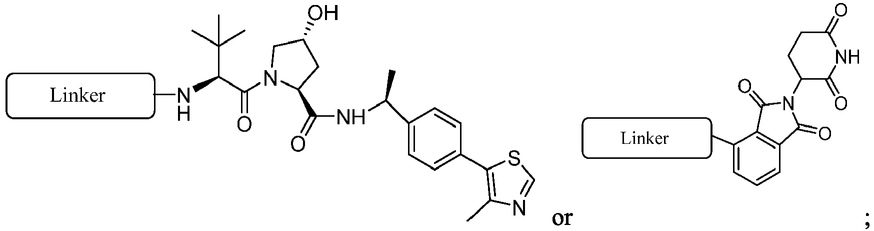
wherein the linker is a C1-C20 alkylene or polyethylene glycol, or there is no such linker;
each of the above-mentioned C1-C4 alkyl, C2-C4 alkenyl, C2-C4 alkynyl, C3-C7 cycloalkyl, C3-C7 cycloalkenyl or C6-C10 aryl is unsubstituted or substituted, wherein the "substituted" means a substitution with 1, 2, 3, 4 or 5 substituents selected from the group consisting of a halogen, C1-C4 alkyl, C1-C4 alkoxy, C3-C7 cycloalkyl, C3-C7 cycloalkenyl and C6-C10 aryl.

In another preferred example, =-=-= represents a double bond, and the double bond configuration is cis or trans.

In another preferred example, the compound has a structure shown in formula II:

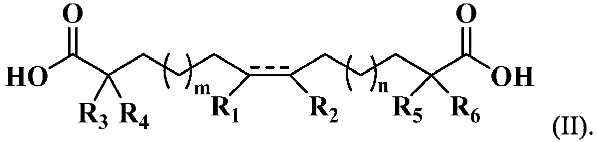

In another preferred example, the compound has a structure shown in formula III:

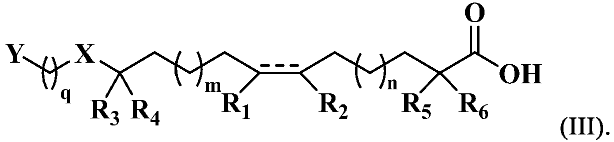

In another preferred example, the compound has a structure shown in formula IV:

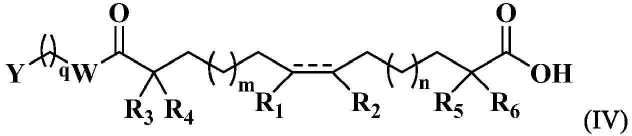

W is absent, -O-, -NH-, -S- or -CH2-.

In another preferred example, R₁ and R₂ are each independently H, methyl, ethyl, propyl, vinyl, propenyl, ethynyl, propynyl, C3-C6 cycloalkyl, C3-C6 cycloalkenyl or C6-C10 aryl; or, R₁ and R₂ together with the attached carbons form a saturated 3-6 membered ring, or a 3-6 membered ring containing one unsaturated double bond.

In another preferred example, R₁ and R₂ are each independently H, C1-C4 alkyl, C2-C4 alkenyl, C2-C4 alkynyl, C3-C7 cycloalkyl, C3-C7 cycloalkenyl or C6-C10 aryl;
the above-mentioned C1-C4 alkyl, C2-C4 alkenyl, C2-C4 alkynyl, C3-C7 cycloalkyl, C3-C7 cycloalkenyl or C6-C10 aryl is unsubstituted or substituted, wherein the "substituted" means substitution with 1, 2, 3, 4 or 5 substituents selected from the group consisting of: halogen, C1-C4 alkyl, C1-C4 alkoxy, C3-C7 cycloalkyl, C3-C7 cycloalkenyl, C6-C10 aryl, and -CO-(5-7 membered heterocyclyl)-(5-7 membered heteroaryl); the above group is optionally further substituted with a substituent selected from the following group: a C6-C10 aryl, halogenated C6-C10 aryl, carboxyl substituted C6-C10 aryl, C1-C4 alkyl substituted C6-C10 aryl, C1-C4 haloalkyl substituted C6-C10 aryl.

In another preferred example, R₁ and R₂ are each independently H, C1-C4 alkyl, C6-C7 cycloalkenyl or C6-C10 aryl; the above-mentioned C1-C4 alkyl, C6-C7 cycloalkenyl or C6-C10 aryl is unsubstituted or substituted, wherein the "substituted" means a substitution with 1, 2, or 3 substituents selected from the group consisting of: a halogen, C1-C4 alkyl, C1-C4 alkoxy, C5-C6 cycloalkyl, C3-C7 cycloalkenyl, C6-C10 aryl, and -CO-(6 membered heterocyclyl containing N)-(6 membered heteroaryl); the above group is optionally further substituted with a substituent selected from the following group: a C6-C10 aryl, halogenated C6-C10 aryl, carboxyl substituted C6-C10 aryl, C1-C4 alkyl substituted C6-C10 aryl, C1-C4 haloalkyl substituted C6-C10 aryl.

In another preferred example, R₃ and R₄ are each independently H, methyl, ethyl, propyl, vinyl, propenyl, ethynyl, propynyl, C3-C6 cycloalkyl, C3-C6 cycloalkenyl or C6-C10 aryl; or, R₃ and R₄ together with the attached carbon form a saturated 3-6 membered ring, or a 3-6 membered ring containing one unsaturated double bond.

In another preferred example, R₅ and R₆ are each independently H, methyl, ethyl, propyl, vinyl, propenyl, ethynyl, propynyl, C3-C6 cycloalkyl, C3-C6 cycloalkenyl or C6-C10 aryl; or, R₅ and R₆ together with the attached carbon form a saturated 3-6 membered ring, or a 3-6 membered ring containing one unsaturated double bond.

In another preferred example, m and n are each independently 1, 2, 3, 4 or 5.

In another preferred example, q is 0, 1, 2, 3 or 4.

In another preferred example, R₁ and R₂ together with the attached carbons form a saturated four-membered ring, a saturated five-membered ring, a saturated six-membered ring, a four-membered ring containing one unsaturated double bond, a five-membered ring containing one unsaturated double bond, or a six-membered ring containing one unsaturated double bond.

In another preferred example, R₃ and R₃ together with the attached carbon form a saturated four-membered ring, a saturated five-membered ring, a saturated six-membered ring, a four-membered ring containing one unsaturated double bond, a five-membered ring containing one unsaturated double bond, or a six-membered ring containing one unsaturated double bond.

In another preferred example, R₅ and R₆ together with the attached carbon form a saturated four-membered ring, a saturated five-membered ring, a saturated six-membered ring, a four-membered ring containing one unsaturated double bond, a five-membered ring containing one unsaturated double bond, or a six-membered ring containing one unsaturated double bond.

In another preferred example, Y is H, methyl, ethyl, propyl, benzene ring, naphthalene ring, methoxy, ethoxy, adamantyl, or an aromatic ring (benzene or naphthalene ring) substituted by p halogens, wherein p is 1, 2, 3, 4, 5, preferably 1 or 2, and said halogen is F, Cl, Br or I; or Y is a structural fragment with acetyl coenzyme A synthase 2 (ACSS2) inhibitory activity: 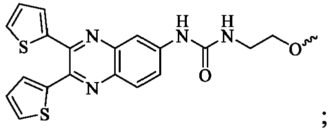
or Y is the following structural fragment with the histone methyltransferase EZH2 inhibitory activity, so that the compound has both ACLY and EZH2 inhibitory activity: 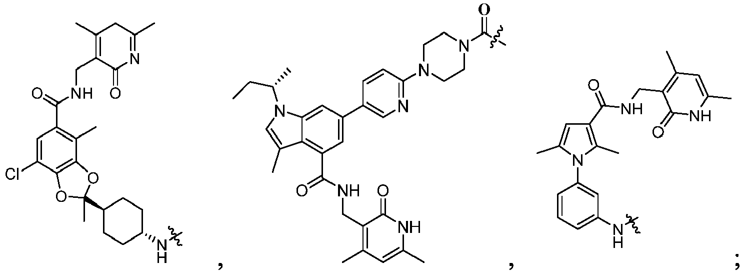
or Y is a structure containing E3 ligase ligand:

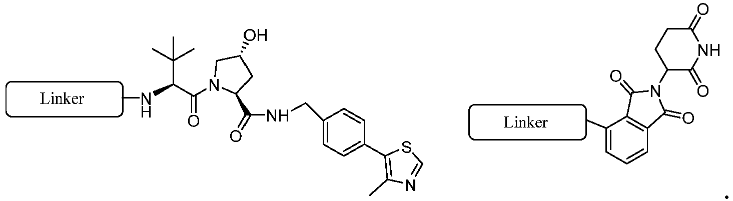

In another preferred example, the compound is selected from M1-M40.

The second aspect of the present invention provides a pharmaceutical composition of compound having ACLY activity comprising the compound of the general formula (I) according to the first aspect, or the stereoisomer, enantiomer, diastereoisomer, racemate, or the pharmaceutically acceptable salt thereof; and a pharmaceutically acceptable carrier.

The dosage forms of the pharmaceutical compositions described herein may be varied, including, but not limited to: a tablet, capsules, granule, syrup, solution, suspension, or aerosol.

"Pharmaceutically acceptable carrier" means: one or more compatible solid or liquid fillers or gel substances which are suitable for human use and which must be of sufficient purity and sufficiently low toxicity. "Compatibility" herein refers to the ability of the components of the composition to be admixed with the compounds of the invention and with each other without appreciably reducing the efficacy of the active ingredient. Examples of pharmaceutically acceptable carriers are cellulose and derivatives thereof (e.g., sodium carboxymethylcellulose, sodium ethylcellulose, cellulose acetate, etc.), gelatin, talc, solid lubricants (e.g., stearic acid, magnesium stearate), calcium sulfate, vegetable oils (e.g., soybean oil, sesame oil, peanut oil, olive oil, etc.), polyols (e.g., propylene glycol, glycerol, mannitol, sorbitol, etc.), emulsifying agents (e.g., Tween^{®}), Wetting agent (e.g. sodium dodecyl sulfate), coloring agent, flavoring agent, stabilizer, antioxidant, preservative, non-hot raw water and the like.

The third aspect of the present invention provides a use of the compound of the general formula (I) according to the first aspect, or the stereoisomer, enantiomer, diastereoisomer, racemate, or pharmaceutically acceptable salt thereof, or the pharmaceutical composition according to the second aspect, as an ACLY inhibitor; in the preparation of an ACLY inhibitor; or in the preparation of a medication for the prevention and/or treatment of metabolic diseases or cancer.

In another preferred example, the metabolic disease is selected from the following group: hyperlipidemia, atherosclerosis, non-alcoholic fatty liver disease, diabetes.

In another preferred example, the cancer is selected from the following group: lung cancer, pancreatic cancer, breast cancer, ovarian cancer, liver cancer, intestinal cancer, brain cancer, acute myeloid leukemia.

The fourth aspect of the present invention provides a therapeutic method, in which compounds having ACLY inhibitory activity are used in combination with other anti-cancer drugs (therapeutics).

It should be understood that each of the above technical features of the invention and each of the technical features specifically described below (e.g., examples) may be combined with each other within the scope of the invention to constitute new or preferred technical solutions. The various features disclosed in the specification may be replaced by any alternative features that provide the same, equal or similar purpose. Due to the limitation of space, they will not be repeated herein.

### Detailed description of the invention

Based on extensive and intensive studies, the inventor of the present application have developed a long-chain compound having ACLY inhibitory activity, which shows very good ACLY inhibitory activity in many kinds of cancer cells such as A549, H358, ASPC-1, MIA-PACA, MCF-7, etc., reduces the level of histone acetylation, inhibits the proliferation of cancer cells themselves, and has good potential for development as a therapeutic drug for cancer or metabolic diseases. On this basis, the present invention has been completed.

### Term

In the present invention, unless otherwise specified, the terms used have their ordinary meanings known to those skilled in the art.

In the present invention, the term "C₁-C₄" means there are 1, 2, 3 or 4 carbon atoms. The term "C₃-C₇" means there are 3, 4, 5, 6 or 7 carbon atoms, and so on.

In the present invention, the term "alkyl" refers to a saturated linear or branched hydrocarbon moiety. For example, the term "C1-C4 alkyl" refers to a linear or branched alkyl having 1 to 4 carbon atoms, including, but not limited to, methyl, ethyl, n-propyl, isopropyl, n-butyl, isobutyl, sec-butyl, and tert-butyl.

In the present invention, the term "alkenyl" refers to a linear or branched hydrocarbon moiety containing at least one double bond. For example, the term "C2-C4 alkenyl" refers to a linear or branched hydrocarbon group having 2 to 4 carbon atoms and containing one double bond, including, but not limited to, vinyl, propenyl, butenyl, and isobutenyl.

In the present invention, the term "alkynyl" refers to a linear or branched alkynyl containing one triple bond, including, but not limited to, ethynyl, propynyl, butynyl, isobutynyl, etc.

In the present invention, the term "cycloalkyl" refers to a saturated cyclic hydrocarbon moiety. For example, the term "C3-C7 cycloalkyl" refers to a cyclic alkyl group having 3 to 7 carbon atoms in the ring, including, but not limited to, cyclopropyl, cyclobutyl, cyclopentyl, cyclohexyl, etc.

In the present invention, the term "cycloalkenyl" refers to a cyclic hydrocarbon moiety containing at least one double bond. For example, the term "C3-C7 cycloalkenyl" refers to a cyclic alkenyl having 3 to 7 carbon atoms in the ring, including, but not limited to, cyclopropenyl, cyclobutenyl, cyclopentenyl, cyclohexenyl, and the like.

In the present invention, the term "aryl" denotes a hydrocarbon moiety containing one or more aromatic rings. For example, the term "C6-C10 aryl" refers to an aromatic ring group with 6 to 10 carbon atoms, such as phenyl, naphthyl, that does not contain heteroatoms on the ring.

In the present invention, the term "heterocyclyl" denotes a saturated or unsaturated, nonaromatic cyclic group comprising at least one (e.g. 1, 2, 3 or 4) cyclic heteroatom (e.g. N, O or S), e.g. tetrahydropyridyl, piperazinyl, pyrrolinyl, dihydropyridyl, dihydrofuryl, dihydrothienyl, morpholinyl.

In the present invention, the term "heteroaryl" denotes an aromatic cyclic group comprising at least one (e.g., 1, 2, 3 or 4) cyclic heteroatom (e.g., N, O or S), e.g., furyl, pyrrolyl, thienyl, oxazolyl, imidazolyl, thiazolyl, pyridinyl, quinolinyl, isoquinolinyl, indolyl, pyrimidyl, pyranyl.

The pharmaceutically acceptable salt of the present invention may be a salt formed by an anion and a positively charged group on the compound of formula I. Suitable anions are chloride ion, bromine ion, iodine ion, sulfate, nitrate, phosphate, citrate, methanesulfonate, trifluoroacetate, acetate, malate, tosylate, tartrate, fumarate, glutamate, glucuronate, lactate, glutarate or maleate. Similarly, the salt can be formed from a cation with negatively charged group on compounds of formula I. Suitable cations include sodium, potassium, magnesium, calcium and ammonium ions, such as tetramethylammonium ion.

### Preparation method

The compounds having general formulas I, II, III, and IV of the present invention can be synthesized by the following routes. 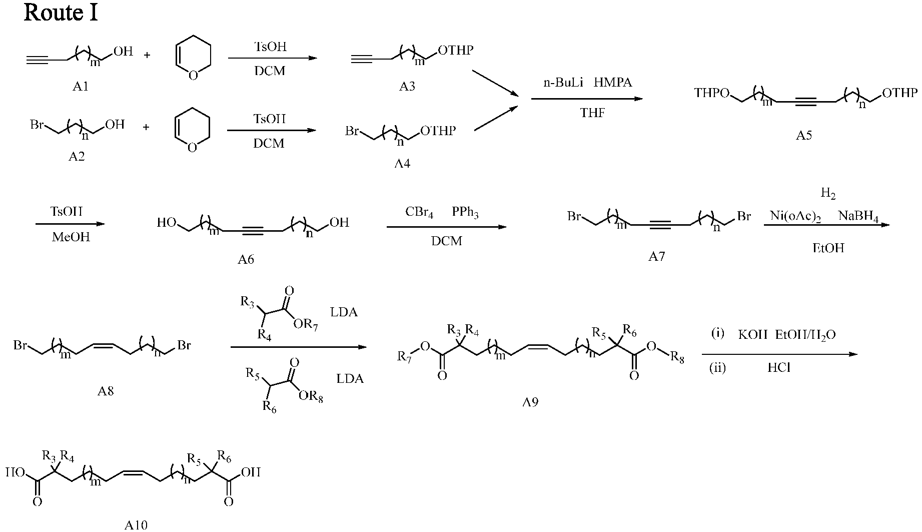
(1) 1-alkynyl alcohol compound Al and excess 3,4-dihydro-2H-pyran (DHP) are catalyzed with p-toluenesulfonic acid (TsOH) to produce the intermediate A3; 1-hydroxybromide and excess 3,4-dihydro-2H-pyran (DHP) are catalyzed with p-toluenesulfonic acid (TsOH) to produce the intermediate A4.
(2) Intermediates A3 and A4 are reacted to produce intermediate A5 in the presence of n-butyl lithium (n-BuLi) and hexamethylphosphorotriamine (HMPA);
(3) Intermediate A5 is catalyzed by TsOH in methanol to remove the protecting group to generate intermediate A6.
(4) Intermediate A6 is reacted with carbon tetrabromide and triphenylphosphine (PPh3) in an Appel reaction to generate intermediate A7.
(5) A7 is converted to A8 with nickel acetate, sodium borohydride and ethylenediamine under hydrogen.
(6)A8 is converted to A9 with an ester having active hydrogen at the α-position under lithium diisopropylammonium (LDA).
(7) A9 is hydrolyzed in the presence of potassium hydroxide and further acidified to give A10.

R₃, R₄, R₅, R₆, m and n are defined in forluma I; R₇ and R₈ are independently methyl or ethyl. 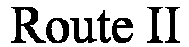 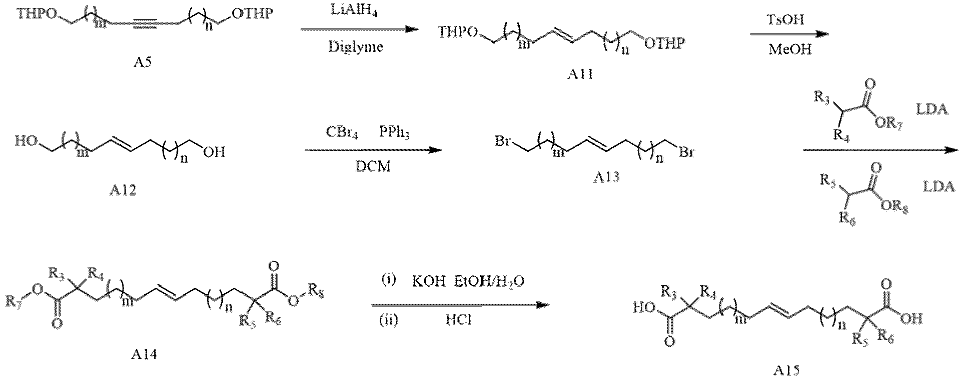
(1) Intermediate A15 is reduced to intermediate A11 containing a trans double bond by lithium aluminum tetrahydrogen (LiAlH₄) at 180 °C.
(2) Intermediate A11 is catalyzed by TsOH to remove protective groups in methanol and generate intermediate A12.
(3) Intermediate A12 undergoes Appel reaction with carbon tetrabromide and triphenylphosphate (PPh₃) to form intermediate A13.
(4) Intermediate A13 and an ester having active hydrogen at the α-position are reacted to generate A14 under the conditions of diisopropylamino lithium.
(5) A14 undergoes hydrolysis in the presence of potassium hydroxide, further acidification to obtain A15. 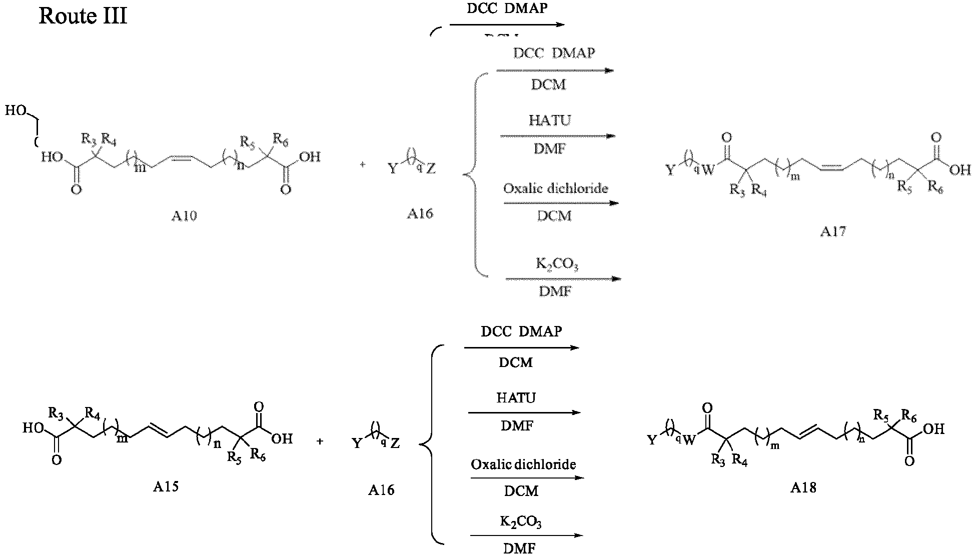
   (1) Intermediate A15 is reduced to intermediate A11 containing a trans double bond by lithium aluminum tetrahydrogen (LiAlH4) at 140 °C.
   (2) Compound A10 and compound A16 undergo a condensation reaction under the conditions of dicyclohexylcarbodiimide (DCC) and 4-dimethylaminopyridine (DMAP) to form compound A17, or undergo a condensation reaction under the conditions of condensation agent N,N,N',N'-tetramethyl-O-(7-azabenzotriazol-1-yl)hexafluorophosphourea (HATU) to form compound A17; or A10 is converted to acyl chloride by oxalyl chloride and then converted to A17 with A16, or A10 and A16 form A17 with potassium carbonate as a base; Compound A15 and compound A16 undergo condensation reaction under the conditions of dicyclohexylcarbodiimide (DCC) and 4-dimethylaminopyridine (DMAP) to form compound A18, or undergo condensation reaction under the conditions of condensation agent N,N,N',N'-tetramethyl-O-(7-azabenzotriazol-1-yl)hexafluorophosphourea (HATU) to form compound A18; or A15 is converted to acyl chloride by oxalyl chloride and then converted to A18 with A16, or A15 and A16 form A18 with potassium carbonate as a base.

R₃, R₄, R₅, R₆, m, n, Y and W are defined as formula IV; R₇ and R₈ are each independently methyl or ethyl, and in addition, Z in A16 is a hydroxyl or halogen. 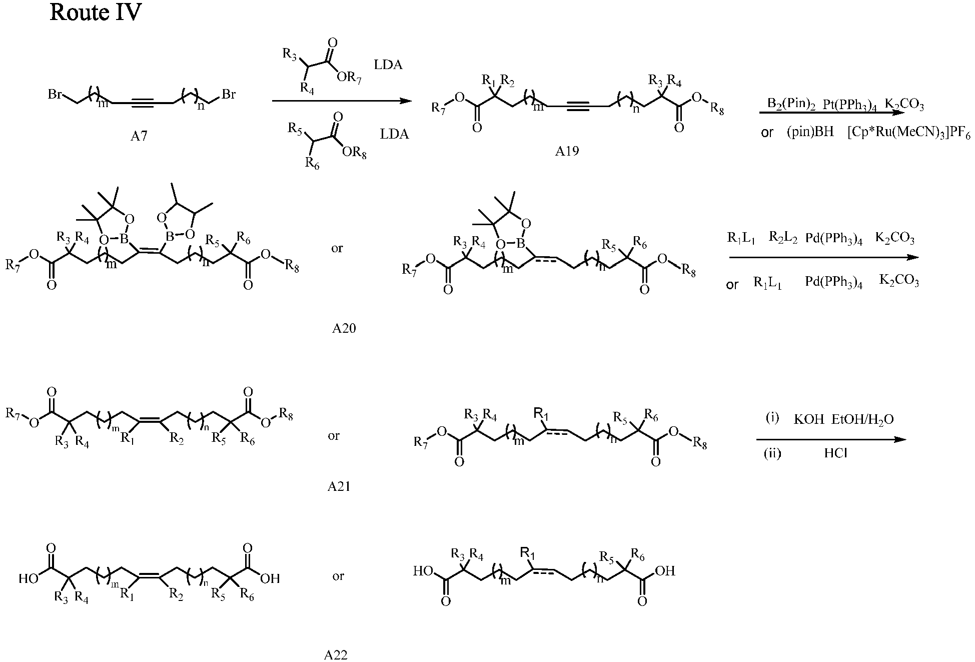
(1) Intermediate A117 and an ester having active hydrogen at α position are reacted to generate intermediate A19 under the conditions of diisopropylamino lithium (LDA).
(2) A19 is reacted with pinacol biborate under the catalysis of tetratriphenylphosphatin, or with pinacol borane under the catalysis of tris (acetonitrile) cyclopentadienyl hexafluorophosphate ruthenium to produce A20.
(3) A20 undergoes coupling reaction with R₁L₁ or R₂L₂ under the catalysis of tetratriphenylphosphopalladium to obtain compound A21.
(4) A21 undergoes hydrolysis under alkaline conditions and undergoes acidification to obtain compound A22.

R₃, R₄, R₅, R₆, m and n are defined as formula I. R₇ and R₈ are each independently methyl or ethyl, L₁ and L₂ are each independently halogen, i.e. F, Cl, Br or I.

The intermediate A7 in route 1 can also be synthesized using route V as follows. 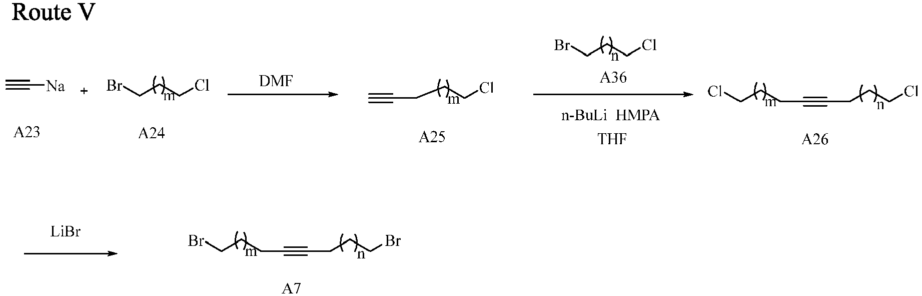
(1) Sodium acetylene suspended in xylene and dihalogenate A34 are reacted in DMF to form intermediate A35.
(2) A35 is subjected to hydrogen extraction via n-butyllithium in the presence of hexamethylphosphorotriamine (HMPA) in the solvent tetrahydrofuran (THF) and then reacted with dihalide A36 to produce dichloride A37.
(3) Intermediate A37 is refluxed with lithium bromide in organic solvent to produce A7.

The present invention will be further described below in conjunction with specific examples. It should be understood that these examples are only used to illustrate the invention and are not intended to limit the scope of the invention. Experimental methods without specifying specific conditions in the following examples are usually carried out according to conventional conditions (such as the conditions described in Sambrook et al., Molecular Cloning: Laboratory Manual (New York: Cold Spring Harbor Laboratory Press, 1989)) or according to manufacturing conditions recommended by the manufacturer. Unless otherwise stated, percentages and parts are by weight.

Unless otherwise defined, all technical and scientific terms used herein have the same meaning as familiar to one skilled in the art. In addition, any methods and materials similar or equivalent to those described herein can be used in the method of the present invention. The preferred implementation methods and materials described herein are for demonstration purposes only.

In the examples described in the present invention, NMR is measured using the Brooke 400M instrument, and NMR calibration: δ H7.26ppm (CDC₁₃), 2.50ppm (DMSO-d6), 2.05ppm (Acetone-d6); mass spectrometry is performed using Agilent 1200 Quadrupole LC/MS liquid chromatography-mass spectrometry; TLC thin layer chromatography silica gel plate is produced by Shandong Yantai Huiyou Silicone Development Co., Ltd., model HSGF 254; the normal phase column chromatography silica gel used for compound purification is produced by the Qingdao Marine Chemical Plant Branch in Shandong Province, with the model zcx-11, 200-300 mesh. Other commonly used commercial reagents are provided by Shanghai Reagent Company.

### Example 1 Synthesis of M1

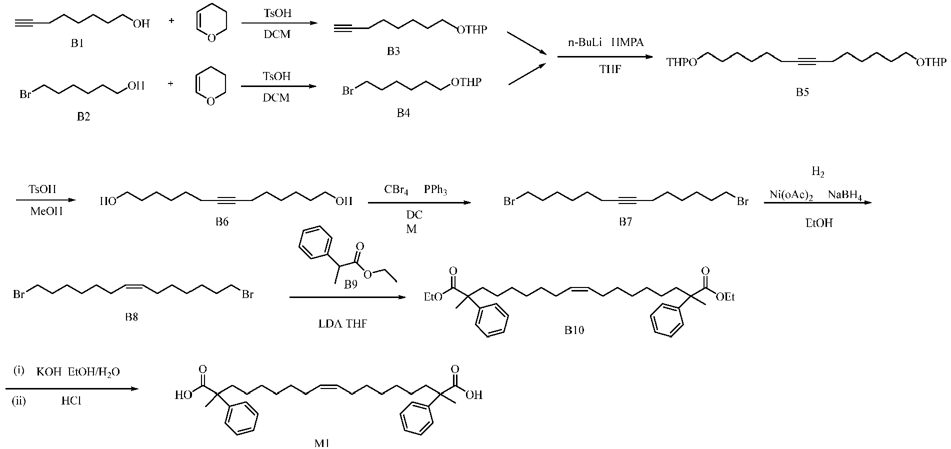

### (1) Preparation of compounds B3 and B4

Under nitrogen protection, 4.6g of compound B1 (36.0mmol) was dissolved in 40ml of dichloromethane (DCM), 1.2g of p-methylbenzenesulfonic acid (TSOH, 7.2mmol) was added, and the mixture was cooled in an ice water bath. 6.1g of 3,4-dihydro-2H-pyran was added dropwise and reacted overnight at room temperature. TLC monitoring showed the reaction was complete, and then the mixture was diluted with 80ml of DCM, washed twice with water, washed once with saturated salt water, dried with anhydrous sodium sulfate, concentrated, and passed through the column with PE: EA=50:1 to obtain product B3 7.1g, with a yield of 95%.

Under nitrogen protection, 5.0g of compound B2 (27.6mmol) was dissolved in 40ml of dichloromethane, 951mg of p-methylbenzenesulfonic acid TSOH (5.5mmol) was added and the mixture was cooled in an ice water bath. 4.6g of 3,4-dihydro-2H-pyran was added dropwise, and reacted overnight at room temperature. TLC monitoring showed the reaction was complete, and then the mixture was diluted with 80ml of DCM, washed twice with water, washed once with saturated salt water, dried with anhydrous sodium sulfate, concentrated, and passed through the column with PE: EA=50:1 to obtain product B4, 5.9g, a colorless oily substance with a yield of 69%.

### (2) Preparation of compound B5

2.0g of compound B3 (9.4mmol) was dissolved in 20ml of dried tetrahydrofuran (THF), protected with nitrogen gas, ventilated three times, and cooled at -78 ° C, then 4.7ml of n-butyl lithium (2.4M 11.3mmol) was added dropwise and 4.0ml of hexamethylphosphotriamine (HMPA) was added and reacted at -78 °C for 30 minutes. 3.0g of compound B4 (11.3mmol) was dissolved in 8ml of dried tetrahydrofuran and added dropwise to the reaction flask. The mixture was reacted overnight at -78 °C to room temperature. TLC monitoring showed the reaction was complete, and the mixture was added with 80ml of saturated ammonium chloride solution and extracted three times with ethyl acetate. The combined organic phases was washed once with saturated saline, dried with anhydrous sodium sulfate, concentrated, and passed the column with PE: EA=10:1 to obtain product B5, 3.2g, a colorless oily substance with a yield of 84%.

### (3) Preparation of compound B6

3.2g of compound B5 (7.9mmol) was dissolved in 30ml of methanol, 136mg (0.79mmol) of p-methylbenzenesulfonic acid TsOH was and reacted at room temperature for 4 hours. TLC monitoring showed the reaction was complete, the mixture was subjected to rotary evaporation to remove methanol, added with 60 ml of ethyl acetate, washed three times with water and once with saturated saline, dried with anhydrous sodium sulfate, concentrated, and passed through the column (PE: EA=3:1) to obtain product B6, 1.3g, white solid, 73% yield.

### (4) Preparation of compound B7

1.9g B6 (8.4mmol) and 5.6g carbon tetrabromide (16.8mmol) were dissolved in 20ml of dry chloromethane, protected by nitrogen, and cooled at 0°C. 4.4g triphenylphosphine (16.8mmol) was dissolved in dry dichloromethane, and added dropwise to the above reaction flask, and the reaction was carried out at room temperature for 2h, and TLC monitoring showed that the reaction was complete. Most of the solvent was spun off, and 80 ml of diethyl ether was added slowly to precipitate a large amount of solid. The solid was removed by diatomaceous earth filtration and washed by diethyl ether, the filtrate was concentrated, and passed through the column (PE: EA=50:1) to obtain product B7, 2.8 g, colorless oil, yield 95%.

### (5) Preparation of compound B8

477mg of nickel acetate (1.9mmol) was suspended in 10ml of ethanol, and sodium borohydride 72mg (1.9mmol) was added. The solution turned from light green to black. The hydrogen balloon was usded, and the gas was exchanged for several times. 0.58ml of ethylenediamine was added, and 1.4g of B7 solution in ethanol was added dropwise, and the reaction was carried out for 3 hours at room temperature. TLC monitoring showed the reaction was complete, 40ml of ethyl acetate was added to dilute the reaction mixture, and the solid was filtered by diatomaceous earth. The filtrate was concentrated, and passed through the column (PE: EA=50:1) to obtain product, 900mg, colorless oil, yield 67%.

### (6) Preparation of compound B10

150 mg B8 (0.4 mmol) and 453 mg B9 (2.5 mmol) were dissolved in 8 ml of dry tetrahydrofuran and protected by nitrogen. The gas was exchanges and the mixture was cooled at -78 °C. 1.3 ml of diisopropylammonium lithium LDA (2.0 M 2.6 mmol) was added dropwise, and the solution was changed from colorless to orange, and the reaction was carried out at -78 °C to room temperature overnight. TLC monitoring showed the reaction was complete. The mixture was added with 40 ml of saturated ammonium chloride solution, and extracted with ethyl acetate three times. The combined organic phases was washed once with saturated saline, dried with anhydrous sodium sulfate, concentrated and passed through the column (PE: EA=20:1) to obtain product B10, 200mg, colorless oil, yield 77%.

### (7) Preparation of compound M1

180mg of B10 (0.28mmol) was dissolved in 8ml of ethanol. 180mg of potassium hydroxide was dissolved in 2ml of water, added to the above reaction bottle, and subjected to reflux reaction overnight. TLC monitoring showed the reaction was complete and then ethanol was removed by rotary evaporation. The residue was diluted with 16ml of water, adjusted pH to about 2 with 1M hydrochloric acid to precipitate solid, then extract with ethyl acetate three times, washed with saturated saline, dried with anhydrous sodium sulfate, concentrated, and passed through the column (PE: EA=2:1) to obtain product M1 110mg, a slightly yellow solid with a yield of 80%. 1H NMR (400 MHz, CDCl3) δ 7.42 - 7.28 (m, 8H), 7.27 - 7.19 (m, 2H), 5.41 - 5.25 (m, 2H), 2.21 - 2.05 (m, 2H), 2.05 - 1.95 (m, 4H), 1.92 - 1.80 (m, 2H), 1.54 (s, 3H), 1.51 (s, 3H), 1.42 - 1.02 (m, 16H).

By replacing different substrates and using a synthesis route similar to M1, the following compounds were obtained.

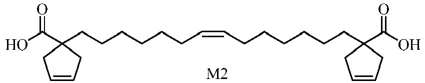

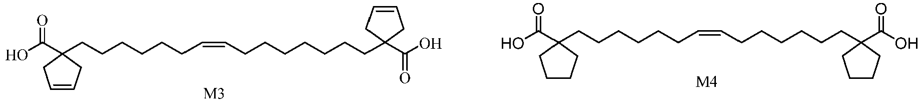

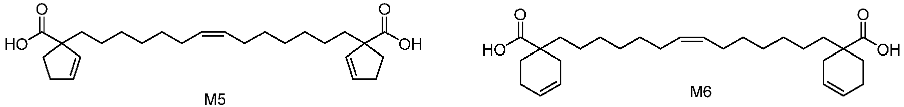

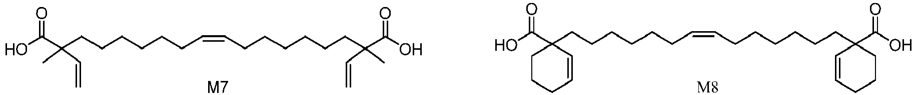

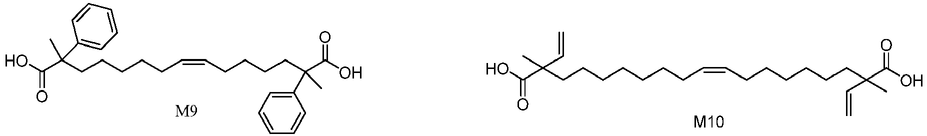

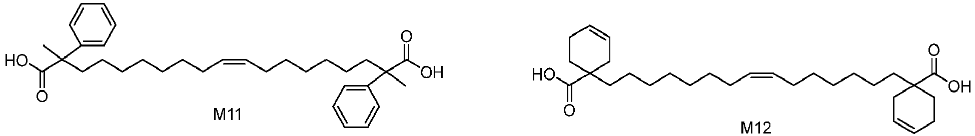

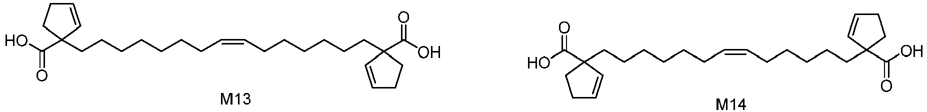

Compound M2: 1H NMR (400 MHz, CDCl3) δ 5.63 - 5.58 (m, 4H), 5.36 - 5.30 (m, 2H), 2.92 (d, J = 15.1 Hz, 4H), 2.29 (d, J = 15.1 Hz, 4H), 2.06 - 1.96 (m, 4H), 1.75 - 1.65 (m, 4H), 1.31 - 1.21 (m, 16H).

Compound M3: 1H NMR (400 MHz, CDCl3) δ 5.66 - 5.55 (m, 4H), 5.39 - 5.26 (m, 2H), 2.91 (d, J = 16.1 Hz, 4H), 2.30 (d, J = 16.1 Hz, 4H), 2.08 - 1.93 (m, 4H), 1.75 - 1.64 (m, 4H), 1.35 - 1.22 (m, 18H).

Compound M4: 1H NMR (400 MHz, CDCl3) δ 5.40 - 5.25 (m, 2H), 2.22 - 2.07 (m, 4H), 2.07 - 1.95 (m, 4H), 1.70 - 1.53 (m, 13H), 1.51 - 1.41 (m, 4H), 1.37 - 1.14 (m, 17H).

Compound M5: 1H NMR (400 MHz, CDCl3) δ 5.85 - 5.78 (m, 2H), 5.77 - 5.70 (m, 2H), 5.39 - 5.27 (m, 2H), 2.46 - 2.30 (m, 6H), 2.07 - 1.95 (m, 4H), 1.76 (dt, J = 17.4, 6.1 Hz, 4H), 1.64 - 1.47 (m, 2H), 1.29 (d, J = 21.1 Hz, 16H).

Compound M6: 1H NMR (400 MHz, CDCl3) δ 5.73 - 5.55 (m, 4H), 5.39 - 5.21 (m, 2H), 2.54 (d, J = 16.9 Hz, 2H), 2.21 - 2.09 (m, 2H), 2.09 - 1.95 (m, 8H), 1.95 - 1.85 (m, 2H), 1.68 - 1.55 (m, 4H), 1.54 - 1.45 (m, 2H), 1.37 - 1.28 (m, 4H), 1.28 - 1.15 (m, 12H).

Compound M7: 1H NMR (400 MHz, CDCl3) δ 6.14 - 6.01 (m, 2H), 5.39 - 5.28 (m, 2H), 5.18 - 5.08 (m, 4H), 2.08 - 1.94 (m, 4H), 1.84 - 1.68 (m, 2H), 1.58 - 1.44 (m, 2H), 1.39 - 1.11 (m, 22H).

Compound M8: 1H NMR (400 MHz, CDCl3) δ 5.70 - 5.58 (m, 4H), 5.37 - 5.27 (m, 2H), 2.54 (d, J = 16.9 Hz, 2H), 2.23 - 2.09 (m, 2H), 2.08 - 1.95 (m, 8H), 1.94 - 1.84 (m, 2H), 1.71 - 1.54 (m, 4H), 1.54 - 1.43 (m, 2H), 1.38 - 1.13 (m, 17H).

Compound M9: 1H NMR (400 MHz, CDCl3) δ 7.39 - 7.31 (m, 10H), 5.44 - 5.31 (m, 2H), 2.14 - 1.89 (m, 8H), 1.55 - 1.50 (m, 6H), 1.40 - 1.15 (m, 10H).

Compound M10: 1H NMR (400 MHz, CDCl3) δ 6.10 - 5.97 (m, 2H), 5.41 - 5.27 (m, 2H), 5.12 (d, J = 13.9 Hz, 4H), 2.07 - 1.92 (m, 4H), 1.80 - 1.66 (m, 2H), 1.64 - 1.49 (m, 2H), 1.35 - 1.23 (m, 24H).

Compound M11: 1H NMR (400 MHz, CDCl3) δ 7.40 - 7.31 (m, 8H), 7.27 - 7.21 (m, 2H), 5.41 - 5.23 (m, 2H), 2.10 - 1.85 (m, 8H), 1.55 (s, 6H), 1.33 - 1.18 (m, 18H).

Compound M12: 1H NMR (400 MHz, CDCl3) δ 5.75 - 5.56 (m, 4H), 5.41 - 5.24 (m, 2H), 2.53 (d, J = 17.3 Hz, 2H), 2.21 - 2.08 (m, 2H), 2.05 - 1.97 (m, 6H), 1.95 - 1.87 (m, 2H), 1.67 - 1.48 (m, 6H), 1.33 - 1.23 (m, 22H).

Compound M13: 1H NMR (400 MHz, CDCl3) δ 5.85 - 5.78 (m, 2H), 5.78 - 5.69 (m, 2H), 5.40 - 5.24 (m, 2H), 2.48 - 2.29 (m, 6H), 2.09 - 1.89 (m, 5H), 1.87 - 1.67 (m, 4H), 1.66 - 1.52 (m, 2H), 1.43 - 1.13 (m, 18H).

Compound M14: 1H NMR (400 MHz, CDCl3) δ 5.86 - 5.78 (m, 2H), 5.77 - 5.69 (m, 2H), 5.39 - 5.28 (m, 2H), 2.46 - 2.32 (m, 6H), 2.06 - 1.93 (m, 4H), 1.83 - 1.71 (m, 4H), 1.64 - 1.55 (m, 2H), 1.32 - 1.24 (m, 14H).

### Example 2 Synthesis of compound M15

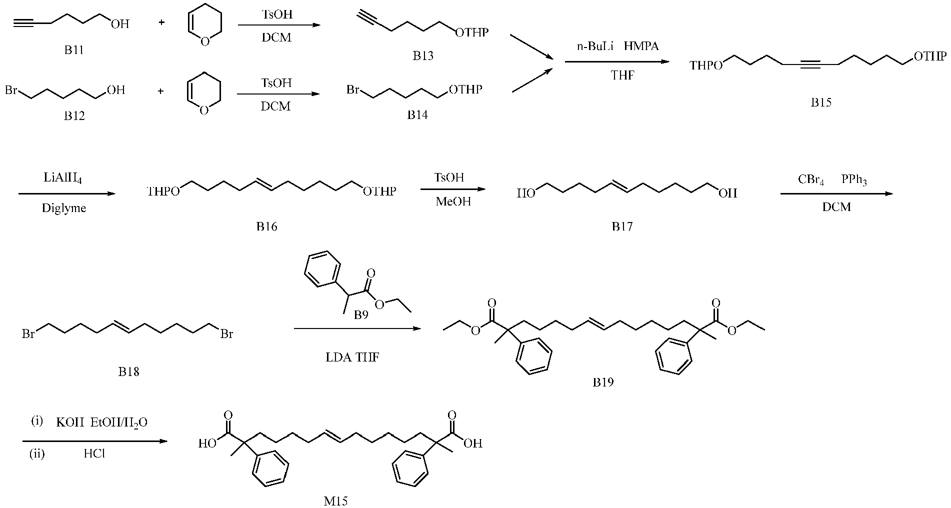

### (1) Preparation of compounds B13 and B14

Under the protection of nitrogen, 4.1 g of compound B11 (41.2 mmol) was dissolved in 30 ml of dichloromethane (DCM), 1.5 g of p-toluenesulfonic acid TSOH (8.2 mmol) was added, cooled in ice-water bath, and 7.4 ml of 3,4-dihydro-2H-pyran was added dropwise, and the reaction was carried out overnight at room temperature. TLC monitoring showed the reaction was complete, and the reaction mixture was diluted by adding 80 ml of DCM, washed with water twice, washed with saturated saline once, dried with anhydrous sodium sulfate, concentrated and passed through the column (PE: EA=50:1) to obtain product B13, 5.5g, colorless oil, yield 39%.

Under the protection of nitrogen, 3.5 g of compound B12 (20.9 mmol) was dissolved in 30 ml of dichloromethane, 794 mg of p-toluenesulfonic acid TSOH (4.2 mmol) was added, cooled in an ice-water bath, and 3.9 ml of 3,4-dihydro-2H-pyran was added dropwise, and the reaction was carried out at room temperature overnight. TLC monitoring showed the reaction was complete, and the reaction mixture was diluted by adding 80 ml of DCM, washed with water twice, washed once with saturated saline, dried with anhydrous sodium sulfate, concentrated, and passed through the column (PE: EA=50:1) to obtain product B14, 6.3g, colorless oil, yield 75%.

### (2) Preparation of compound B15

1.8g compound B13 (9.9 mmol) was dissolved in 20 ml of dry tetrahydrofuran (THF), protected by nitrogen with three gas exchanges, cooled at -78 °C, 7.4 ml of n-butyllithium (1.6 M 11.9 mmol) was added dropwise, 3.0 ml of hexamethylphosphorotriamine (HMPA) was added, and the reaction was carried out at -78 °C for 30 min. 2.5g compound B14 (11.3 mmol) was dissolved in 6 ml of dry tetrahydrofuran, added dropwise to the above reaction flask, and reacted at -78°C to room temperature overnight, TLC monitoring showed the reaction was complete, 50 ml saturated ammonium chloride solution was added, and the mixture was extracted with ethyl acetate three times. The combined organic phases was washed with saturated brine once, dried with anhydrous sodium sulfate, concentrated, and passed through the column (PE: EA=10:1) to obtain product B15, 1.9g, colorless oil, yield 55%.

### (3) Preparation of compound B16

Under the protection of nitrogen, 726 mg of lithium tetrahydroaluminate (18.2 mmol) was dissolved in 10 ml of diethylene glycol dimethyl ether, 800 mg of B15 (2.3 mmol) was dissolved in 4 ml of diethylene glycol dimethyl ether, and added dropwise to the above reaction solution was. The reaction was carried out at 140 °C for 28 hours, and the TLC monitoring showed the reaction was complete. The mixture was diluted with 40 ml of diethyl ether and cooled in an ice-water bath. Sodium sulfate decahydrate was added in batches until there was not bubble. The mixture was filtered by diatomaceous earth to remove solid, washed with anhydrous diethyl ether, dried with anhydrous sodium sulfate, concentrated, and passed through the column (PE: EA=20:1) to obtain product B16 487mg, colorless liquid, yield 61%.

### (4) Preparation of compound B17

1g of B16 (2.8mmol) was dissolved in 20ml of methanol, 100mg of p-toluenesulfonic acid was added and stirred at room temperature for 3 hours. TLC monitoring showed the reaction was complete, the methanol was spun off, 80ml of water was added, the mixture was extracted by ethyl acetate for three times, washed with saturated brine, dried with anhydrous sodium sulfate, concentrated, and passed through the column (PE: EA=3:1) to obtain product B17, 470mg, colorless oil, yield 90%.

### (5) Preparation of compound B18

Under the protection of nitrogen, 470 mg of B18 (2.5 mmol) and 2.5 g of carbon tetrabromide (7.5 mmol) were dissolved in 10 ml of dry dichloromethane, cooled at 0 °C, 1.9 g of triphenylphosphorane (7.5 mmol) was dissolved in dry dichloromethane, and was added dropwise to the above reaction flask, and the reaction was carried out for 2 h at room temperature. TLC monitoring showed that the reaction was complete. 40ml of diethyl ether was added slowly to precipitate a large amount of solid. The solid was removed by diatomaceous earth filtration and washed with diethyl ether. The filtrate was concentrated and passed through the column (PE:EA=50:1) to obtain product, 740mg, colorless oil, yield 95%.

### (6) Preparation of compound B19

Under the protection of nitrogen, 150 mg B18 (0.48 mmol) and 510 mg B9 (2.9 mmol) were dissolved in dry tetrahydrofuran, nitrogen gas was exchanged, cooled at -78 °C, 1.5 ml of lithium diisopropylammonium LDA (2.0 M 3.0 mmol) was slowly added dropwise, the solution was changed from colorless to orange-yellow, and reacted at -78 °C to room temperature overnight. TLC monitoring showed the reaction was complete, 40ml saturated ammonium chloride solution was added, extracted with ethyl acetate three times. The combined organic phases was washed with saturated brine once, dried with anhydrous sodium sulfate, concentrated, and passed through the column (PE: EA=20:1) to obtain product B19, 130mg, yellow oil, yield 54%.

### (7) Preparation of compound M15

130 mg of B19 (0.26 mmol) was dissolved in 8 ml of ethanol and 130 mg of potassium hydroxide was dissolved in 2 ml of water, added to the reaction flask and reacted at reflux overnight. TLC monitoring showed the reaction was complete, ethanol was spun off, the residue was diluted with 20 ml of water, adjusted pH to 2 with 1 M HCl to precipitate a solid, extracted three times with ethyl acetate, and the organic phases were combined, washed with saturated saline, dried with anhydrous sodium sulfate, concentrated, and passed through the column (PE: EA=2:1) to obtain product M23, 80mg, yellow oil, yield 69%. 1H NMR (400 MHz, CDCl3) δ 7.39 - 7.30 (m, 8H), 7.25 - 7.20 (m, 2H), 5.36 - 5.27 (m, 2H), 2.08 - 2.02 (m, 2H), 2.00 - 1.87 (m, 6H), 1.55 (s, 6H), 1.33 - 1.18 (m, 10H).

Using the same synthetic route as M15, the following compounds can be obtained by changing different substrates..

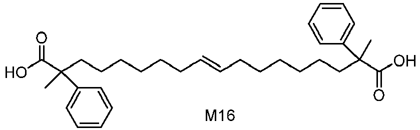

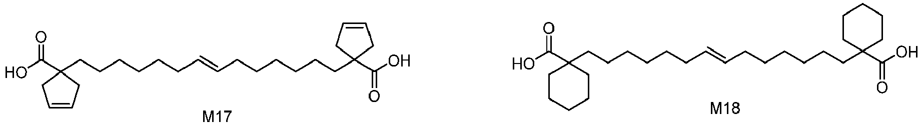

Compound M16: 1H NMR (400 MHz, CDCl3) δ 7.42 - 7.28 (m, 8H), 7.26 - 7.23 (m, 2H), 5.42 - 5.23 (m, 2H), 2.31 - 2.16 (m, 2H), 2.08 - 1.71 (m, 6H), 1.52 (s, 3H), 1.46 (s, 3H), 1.34 - 1.24 (m, 16H).

Compound M17: 1H NMR (400 MHz, CDCl3) δ 5.66 - 5.55 (m, 4H), 5.33 - 5.23 (m, 2H), 2.93 (d, J = 15.7 Hz, 4H), 2.29 (d, J = 15.7 Hz, 4H), 2.03 - 1.91 (m, 4H), 1.76 - 1.64 (m, 4H), 1.33 - 1.19 (m, 16H).

Compound M18: 1H NMR (400 MHz, CDCl3) δ 5.34 - 5.21 (m, 2H), 2.15 - 2.03 (m, 4H), 2.02 - 1.90 (m, 4H), 1.64 - 1.53 (m, 6H), 1.51 - 1.44 (m, 4H), 1.45 - 1.34 (m, 4H), 1.34 - 1.11 (m, 22H).

### Example 3 Synthesis of compound M19

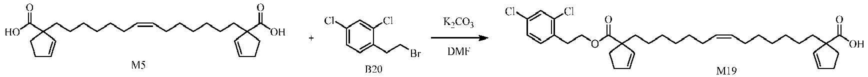

Under the protection of nitrogen, 190 mg of compound M5 (0.46 mmol) was dissolved in 6 ml of DMF, 107 mg of B20 (0.46 mmol) and 126 mg of potassium carbonate (0.92 mmol) were added and the reaction was carried out at room temperature overnight. TLC monitoring showed that a new point was generated. The solvent was spun off and the residue was diluted with 50 ml of water. The pH was adjusted to 2 by 1 M HCl, and the mixture was extracted with ethyl acetate three times, washed with saturated saline, dried with anhydrous sodium sulfate, concentrated, and passed through the column (DCM: MeOH = 20:1) to obtain 100 mg of product, yellowish oil, yield 37%. 1H NMR (400 MHz, CDCl3) δ 7.38 (s, 1H), 7.17 (d, J = 1.0 Hz, 2H), 5.86 - 5.80 (m, 1H), 5.80 - 5.74 (m, 1H), 5.73 - 5.69 (m, 1H), 5.67 - 5.61 (m, 1H), 5.40 - 5.28 (m, 2H), 4.29 (t, J = 6.2 Hz, 2H), 3.05 (t, J = 6.6 Hz, 2H), 2.49 - 2.24 (m, 6H), 2.07 - 1.94 (m, 4H), 1.74 - 1.59 (m, 6H), 1.34 - 1.16 (m, 16H).

By substituting different B20 analogues and using a synthesis method similar to M19, the following compounds can be obtained.

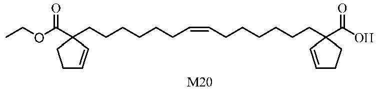

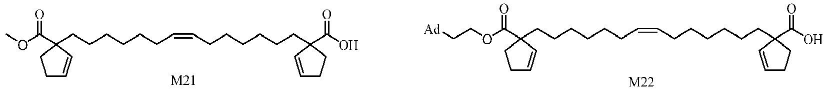

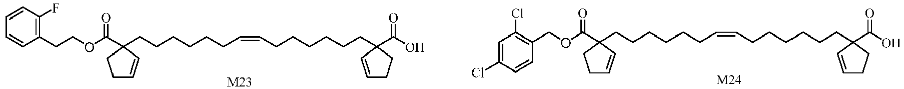

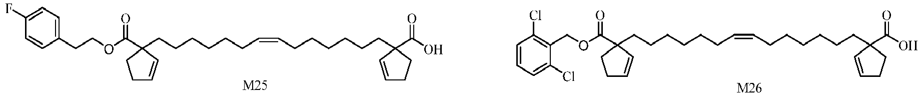

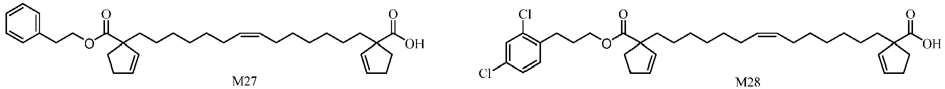

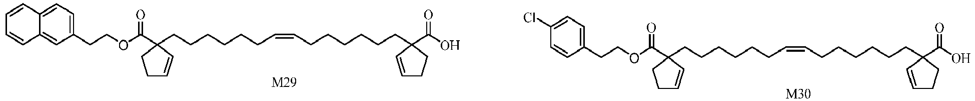

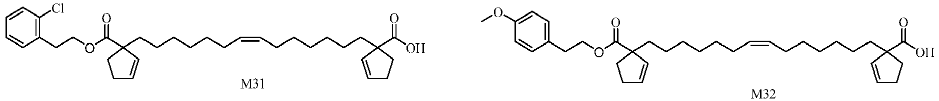

Compound M20: 1H NMR (400 MHz, CDCl3) δ 5.86 - 5.81 (m, 1H), 5.81 - 5.76 (m, 1H), 5.74 - 5.68 (m, 2H), 5.38 - 5.29 (m, 2H), 4.13 (t, J = 7.1, 3.0 Hz, 2H), 2.46 - 2.29 (m, 7H), 2.07 - 1.93 (m, 4H), 1.77 - 1.60 (m, 6H), 1.28 - 1.24 (m, 16H).

Compound M21: 1H NMR (400 MHz, CDCl3) δ 5.85 - 5.77 (m, 2H), 5.75 - 5.67 (m, 2H), 5.38 - 5.30 (m, 2H), 3.67 (s, 3H), 2.48 - 2.30 (m, 6H), 2.05 - 1.93 (m, 4H), 1.81 - 1.55 (m, 6H), 1.34 - 1.23 (m, 16H).

Compound M22: 1H NMR (400 MHz, CDCl3) δ 5.87 - 5.80 (m, 1H), 5.80 - 5.74 (m, 1H), 5.73 - 5.65 (m, 2H), 5.39 - 5.26 (m, 2H), 4.12 (td, J = 7.4, 1.7 Hz, 2H), 2.46 - 2.30 (m, 6H), 2.02 - 1.93 (m, 6H), 1.84 - 1.56 (m, 12H), 1.52 (d, J = 2.0 Hz, 6H), 1.41 (t, J = 7.2 Hz, 2H), 1.27 (d, J = 10.4 Hz, 16H).

Compound M23: 1H NMR (400 MHz, CDCl3) δ 7.24 - 7.16 (m, 2H), 7.09 - 6.98 (m, 2H), 5.86 - 5.80 (m, 1H), 5.78 - 5.73 (m, 1H), 5.73 - 5.68 (m, 1H), 5.66 - 5.61 (m, 1H), 5.39 - 5.28 (m, 2H), 4.29 (td, J = 6.7, 1.8 Hz, 2H), 2.98 (t, J = 6.7 Hz, 2H), 2.48 - 2.26 (m, 6H), 2.05 - 1.93 (m, 4H), 1.82 - 1.47 (m, 6H), 1.32 - 1.10 (m, 16H).

Compound M24: 1H NMR (400 MHz, CDCl3) δ 7.40 (d, J = 1.9 Hz, 1H), 7.33 (d, J = 8.3 Hz, 1H), 7.24 (dd, J = 8.3, 1.9 Hz, 1H), 5.85 - 5.77 (m, 2H), 5.75 - 5.66 (m, 2H), 5.39 - 5.26 (m, 2H), 5.16 (d, J = 2.7 Hz, 2H), 2.50 - 2.27 (m, 7H), 2.04 - 1.95 (m, 4H), 1.84 - 1.70 (m, 5H), 1.63 - 1.55 (m, 2H), 1.30 - 1.19 (m, 16H).

Compound M25: 1H NMR (400 MHz, CDCl3) δ 7.21 - 7.12 (m, 2H), 7.03 - 6.91 (m, 2H), 5.87 - 5.80 (m, 1H), 5.80 - 5.73 (m, 1H), 5.73 - 5.67 (m, 1H), 5.67 - 5.61 (m, 1H), 5.40 - 5.28 (m, 2H), 4.26 (t, J = 6.4 Hz, 2H), 2.90 (t, J = 6.5 Hz, 2H), 2.48 - 2.25 (m, 6H), 2.06 - 1.93 (m, 4H), 1.84 - 1.46 (m, 6H), 1.32 - 1.18 (m, 16H).

Compound M26: 1H NMR (400 MHz, CDCl3) δ 7.34 (d, J = 7.8 Hz, 2H), 7.25 - 7.17 (m, 1H), 5.86 - 5.80 (m, 1H), 5.79 - 5.73 (m, 1H), 5.73 - 5.66 (m, 2H), 5.40 - 5.26 (m, 4H), 2.47 - 2.29 (m, 6H), 2.03 - 1.91 (m, 4H), 1.81 - 1.66 (m, 4H), 1.65 - 1.50 (m, 3H), 1.32 - 1.22 (m, 16H).

Compound M27: 1H NMR (400 MHz, CDCl3) δ 7.33 - 7.26 (m, 2H), 7.25 - 7.18 (m, 3H), 5.85 - 5.80 (m, 1H), 5.79 - 5.74 (m, 1H), 5.73 - 5.68 (m, 1H), 5.68 - 5.63 (m, 1H), 5.39 - 5.28 (m, 2H), 4.29 (td, J = 6.9, 2.9 Hz, 2H), 2.93 (t, J = 6.9 Hz, 2H), 2.50 - 2.26 (m, 6H), 2.07 - 1.92 (m, 4H), 1.80 - 1.50 (m, 6H), 1.32 - 1.19 (m, 16H).

Compound M28: 1H NMR (400 MHz, CDCl3) δ 7.36 (d, J = 1.9 Hz, 1H), 7.20 - 7.10 (m, 2H), 5.86 - 5.77 (m, 2H), 5.74 - 5.67 (m, 2H), 5.38 - 5.27 (m, 2H), 4.10 (t, J = 6.2 Hz, 2H), 2.82 - 2.72 (m, 2H), 2.49 - 2.31 (m, 6H), 2.05 - 1.89 (m, 6H), 1.84 - 1.68 (m, 4H), 1.60 (dd, J = 11.9, 3.5 Hz, 2H), 1.32 - 1.23 (m, 16H).

Compound M29: 1H NMR (400 MHz, CDCl3) δ 7.83 - 7.74 (m, 3H), 7.65 (s, 1H), 7.48 - 7.40 (m, 2H), 7.35 (dd, J = 8.4, 1.5 Hz, 1H), 5.84 - 5.79 (m, 1H), 5.78 - 5.73 (m, 1H), 5.73 - 5.68 (m, 1H), 5.67 - 5.63 (m, 1H), 5.36 - 5.27 (m, 2H), 4.44 - 4.31 (m, 2H), 3.09 (t, J = 6.8 Hz, 2H), 2.43 - 2.28 (m, 6H), 2.04 - 1.91 (m, 5H), 1.81 - 1.66 (m, 4H), 1.62 - 1.49 (m, 2H), 1.29 - 1.11 (m, 16H).

Compound M30: 1H NMR (400 MHz, CDCl3) δ 7.25 (d, J = 5.2 Hz, 2H), 7.14 (d, J = 8.3 Hz, 2H), 5.87 - 5.80 (m, 1H), 5.80 - 5.75 (m, 1H), 5.73 - 5.68 (m, 1H), 5.66 - 5.61 (m, 1H), 5.38 - 5.28 (m, 2H), 4.30 - 4.19 (m, 2H), 2.90 (t, J = 6.7 Hz, 2H), 2.46 - 2.24 (m, 6H), 2.06 - 1.94 (m, 4H), 1.80 - 1.52 (m, 6H), 1.32 - 1.19 (m, 16H).

Compound M31: 1H NMR (400 MHz, CDCl3) δ 7.37 - 7.33 (m, 1H), 7.25 - 7.22 (m, 1H), 7.21 - 7.14 (m, 2H), 5.86 - 5.80 (m, 1H), 5.79 - 5.74 (m, 1H), 5.73 - 5.68 (m, 1H), 5.67 - 5.62 (m, 1H), 5.38 - 5.28 (m, 2H), 4.31 (td, J = 6.8, 2.1 Hz, 2H), 3.08 (t, J = 6.8 Hz, 2H), 2.44 - 2.29 (m, 6H), 2.04 - 1.94 (m, 4H), 1.79 - 1.51 (m, 6H), 1.31 - 1.22 (m, 16H).

Compound M32: 1H NMR (400 MHz, CDCl3) δ 7.13 (d, J = 8.6 Hz, 2H), 6.83 (d, J = 8.6 Hz, 2H), 5.85 - 5.80 (m, 1H), 5.79 - 5.75 (m, 1H), 5.73 - 5.68 (m, 1H), 5.68 - 5.64 (m, 1H), 5.38 - 5.28 (m, 2H), 4.24 (td, J = 7.0, 2.7 Hz, 2H), 3.78 (s, 3H), 2.87 (t, J = 6.9 Hz, 2H), 2.44 - 2.26 (m, 6H), 2.05 - 1.94 (m, 4H), 1.80 - 1.53 (m, 6H), 1.32 - 1.18 (m, 16H).

### Example 4 Synthesis of compound M33

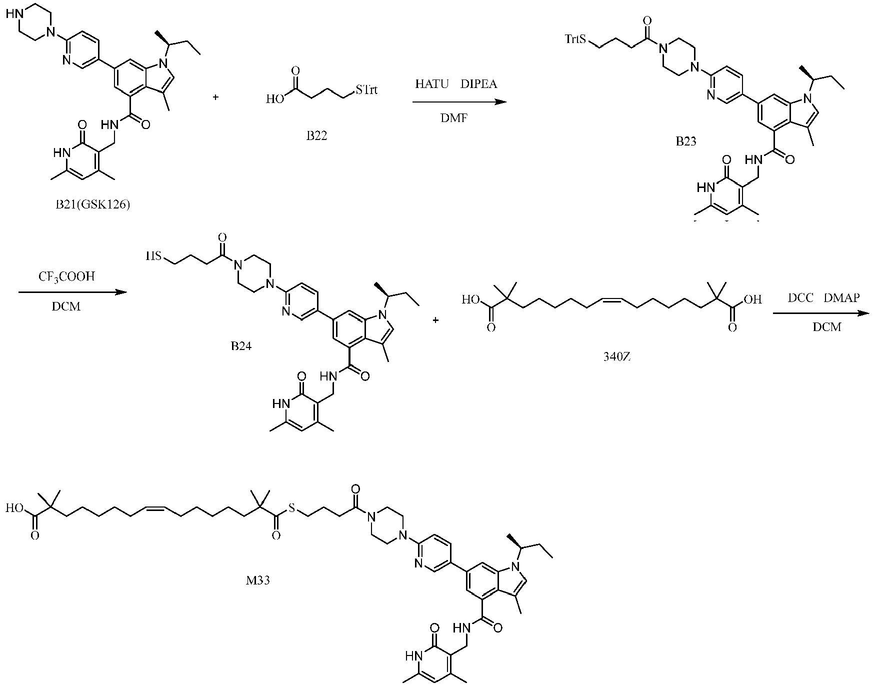

### (1) Preparation of compound B23

Under nitrogen protection, 600 mg of compound B21 (1.1 mmol) and 599 mg of compound B22 (1.7 mmol) were dissolved in 26 ml of dry DMF (N,N-dimethylformamide), and 646 mg of N,N,N',N'-tetramethyl-O-(7-azabenzotriazol-1-yl)hexafluorophosphorourea HATU (1.7 mmol) and N N-diisopropylethylamine DIPEA 4.8 ml were added, the reaction was carried out at room temperature overnight. TLC monitoring showed the reaction was complete, DMF was spun off, and the residue was added with 60 ml of ethyl acetate, washed with water three times, washed with saturated saline once, dried with anhydrous sodium sulfate, concentrated, and passed through the column (DCM: MeOH = 20:1) to obtain product B23 900mg, orange powder solid, yield 94%.

### (2) Preparation of compound B24

Under the protection of nitrogen, 900 mg of B23 was dissolved in 8 ml of dichloromethane, 2.0 ml of trifluoroacetic acid was added, and the reaction was reacted at room temperature for 1 h. TLC monitoring showed that the reaction was complete, and the reaction was quenched by adding 50 ml of saturated sodium bicarbonate, and the reaction mixture was extracted by dichloromethane for several times, concentrated, and passed through the column (DCM: MeOH = 10:1) to obtain product B24, 513mg, white solid, yield 82%.

### (3) Preparation of compound M33

Under the protection of nitrogen, 512 mg B24 (0.8 mmol) and 514 mg M18 (1.5 mmol) were dissolved in 16 ml of dry dichloromethane, cooled at zero degree, 309 mg DCC (1.5 mmol) and 98 mg (0.8 mmol) DMAP were added, and the reaction was carried out at zero degree to room temperature overnight. TLC monitoring showed that the reaction was complete, the solid was removed by filtration, and 80 ml of water was added. The mixture was extracted with DCM three times, washed with saturated saline, concentrated, and passed through the column (DCM: MeOH = 20:1) to obtain product, 318mg, white powder solid, yield 42%. 1H NMR (400 MHz, CDCl3) δ 8.47 (d, J = 2.4 Hz, 1H), 7.80 (dd, J = 7.7, 5.6 Hz, 1H), 7.47 (s, 1H), 7.02 (s, 1H), 6.96 (d, J = 5.5 Hz, 1H), 6.76 - 6.68 (m, 1H), 6.15 (d, J = 7.2 Hz, 1H), 5.35 - 5.26 (m, 3H), 4.60 (d, J = 2.5 Hz, 2H), 4.39 (dd, J = 13.2, 6.5 Hz, 1H), 3.77 (d, J = 3.1 Hz, 2H), 0.83 (dd, J = 8.2, 6.3 Hz, 3H).

The above compound B22 can be synthesized by conventional chemical methods.

Using different substrates and similar synthesis methods as mentioned in route III, the following compounds were synthesized.

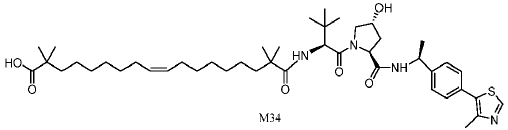

Compound M34: 1H NMR (400 MHz, CDCl3) δ 8.70 (d, J = 4.3 Hz, 1H), 8.67 (s, 1H), 8.40 (d, J = 8.2 Hz, 1H), 7.41 - 7.35 (m, 4H), 6.28 (d, J = 7.8 Hz, 1H), 5.32 (d, J = 16.1 Hz, 2H), 5.11 - 5.03 (m, 1H), 4.75 (t, J = 7.9 Hz, 1H), 4.50 (d, J = 7.4 Hz, 2H), 4.22 (d, J = 11.1 Hz, 1H), 3.55 (d, J = 9.6 Hz, 1H), 2.53 (s, 3H), 2.07 - 1.97 (m, 4H), 1.86 - 1.80 (m, 2H), 1.63 - 1.61 (m, 5H), 1.51 - 1.49 (m, 6H), 1.49 - 1.46 (m, 4H), 1.39 - 1.36 (m, 4H), 1.28 - 1.23 (m, 6H), 1.18 - 1.15 (m, 6H), 1.06 - 1.01 (m, 9H).

### Example 5 Synthesis of compound M35

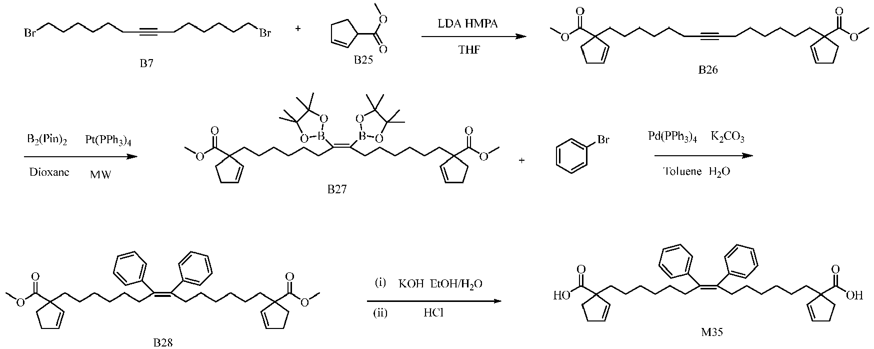

### (1) Preparation of compound B26

Under the protection of nitrogen, 3.0 g B7 (8.5 mmol) was dissolved in 30 ml of dry tetrahydrofuran, 5.9 ml of hexamethylphosphorotriamine HMPA (34 mmol) was added, cooled at -78 °C, with several nitrogen gas exchanges, 17 ml of lithium diisopropylammonium LDA (2.0 M 34 mmol) was added slowly dropwise and the solution was changed from colorless to orange-yellow, and 30 min later, 4.3 g B25 (34.0 mmol) was added slowly dropwise and reacted at -78 °C to room temperature reaction overnight. TLC monitoring showed the reaction was complete, the mixture was added with 80 ml of saturated ammonium chloride and extracted with ethyl acetate extraction three times. The organic phases were combined, washed with saturated saline once, dried with anhydrous sodium sulfate, concentrated, and passed through the column (PE: EA=10:1) to obtain product, 2.3g, colorless oil, yield 61%.

### (2) Preparation of compound B27

400mg B26 (0.93mmol) and 474mg pinalol diborate (1.8mmo) were dissolved in 6ml dioxane, and the catalyst Platinum tetrakis(triphenylphosphine) 111mg (0.09mmol) was added, and the reaction was carried out by microwave at 110°C for one hour, and the solution was changed from yellow color to maroon. TLC monitoring showed that the reaction was complete, the mixture was filtrated to remove the solid and subjected to rotary evaporation to remove solvent, and then passed through the column (PE: EA=10:1) to obtain product, 338mg, colorless oil, yield 52%.

### (3) Preparation of compound B28

338 mg of B27 (0.48 mmol), 228 mg of bromobenzene (1.45 mmol) and 402 mg of potassium carbonate (2.91 mmol) were dissolved in 12 ml of toluene and 1.5 ml of water, nitrogen was exchanged for several times, and 58 mg (0.05 mmol) of catalyst tetrakis(triphenylphosphine)palladium was added, and the reaction was carried out at 90 °C overnight, and the solution was changed from yellow to light green. TLC monitoring showed the reaction was complete, the solid was removed by filtration. The residue was diluted with 60 ml of water, extracted three times with ethyl acetate. The organic phases were combined, washed with saturated saline, dried with anhydrous sodium sulfate, concentrated, and passed through the column to obtain product 188mg, colorless oil, yield 66%.

### (4) Preparation of compound M35

142 mg of B28 (0.24 mmol) was dissolved in 16 ml of ethanol and 2 ml of water, 284 mg of potassium hydroxide was added and the reaction was carried out overnight at 60 °C. TLC monitoring showed the reaction was complete, ethanol was spun off, and the residue was diluted with 30 ml of water, adjusted to acidity by 1 M HCl, and extracted three times with ethyl acetate. The organic phases were combined, washed with saturated saline, dried with anhydrous sodium sulfate, concentrated, and passed through the column to obtain product, 69mg, yellowish powdery solid, yield 51%. 1H NMR (400 MHz, CDCl3) δ 7.07 - 6.90 (m, 10H), 5.86 - 5.78 (m, 2H), 5.77 - 5.70 (m, 2H), 2.56 - 2.46 (m, 4H), 2.46 - 2.30 (m, 7H), 1.81 - 1.69 (m, 4H), 1.64 - 1.52 (m, 2H), 1.35 - 1.25 (m, 16H).

### Example 6 Synthesis of compound M36

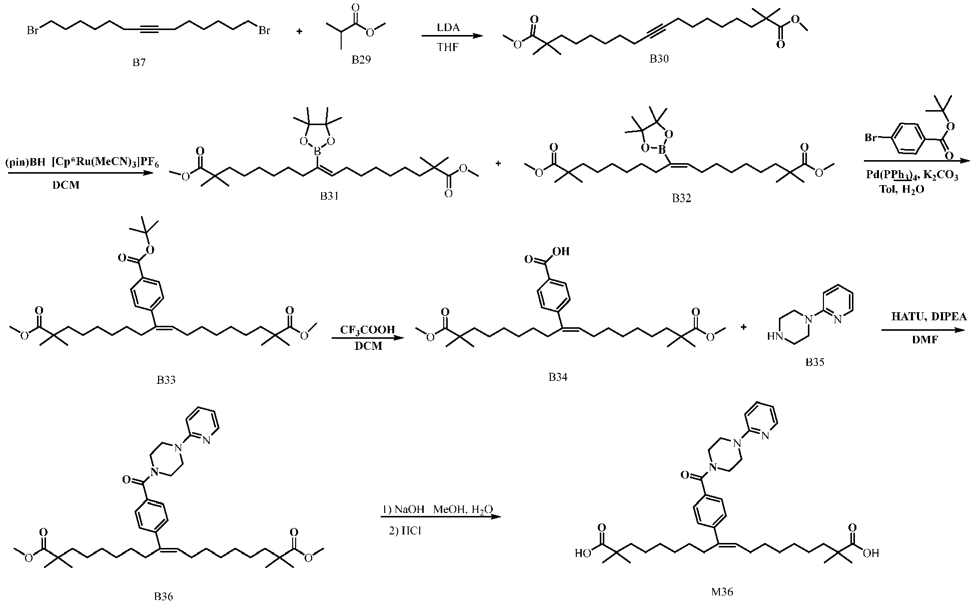

### 1) Preparation of compound B30

Under the protection of nitrogen, 1.6 g of B7 (5 mmol) and 2.04 g of B29 (20 mmol) were dissolved in dry tetrahydrofuran, nitrogen was exchanged for several times, the mixture was cooled at -78 °C, 10 ml of LDA (20 mmol) was slowly dripped into the reaction solution, the reaction solution was changed from colorless to orange, and the reaction was carried out at room temperature at -78 °C overnight. TLC monitoring showed the reaction was complete, the mixture was added with 80ml of saturated ammonium chloride and extracted with ethyl acetate three times. The organic phases were combined, washed with saturated saline once, dried with anhydrous sodium sulfate, concentrated, and passed through the column (PE: EA=20:1) to obtain product 1.2g, colorless oil, yield 61%.

### (2) Preparation of compound B31 and compound B32

Under the protection of nitrogen, 1.2g B30 (3mmol) and 768mg pinacolborane (6mmol) were dissolved in 50ml dichloromethane, nitrogen was exchanged for several times, and 257mg of tris(acetonitrile)cyclopentadienyl ruthenium hexafluorophosphate (0.6mmol) was added in an ice bath at 0°C, and the solution was changed from colorless to brown, and the reaction was carried out at room temperature for 3h. TLC monitoring showed the reaction was complete. The magnetic stirrer was removed, 1.5 g silica gel was added and mixed well and passed the column (dry sampling, PE: EA = 30:1) to obtain 501mg of compound B31, 520mg of compound B32, which are colorless oily compounds, yield 33%.

### 3) Preparation of compound B33

520 mg of B32 (1 mmol) and 514 mg of tert-butyl 4-bromobenzoate (2 mmol) were dissolved in 10 ml of 1,4-dioxane and 2 ml of water, nitrogen was exchanged for several times, and then 828 mg of potassium carbonate (6 mmol) and 231 mg of tetrakis(triphenylphosphine)palladium (0.2 mmol) were added in turn, and the solution turned from colorless to brown, and the reaction was carried out at 80 °C overnight. TLC monitoring showed the reaction was complete, the mixture was cooled to room temperature, diluted by adding 60 ml of ethyl acetate and washed three times with water, washed with saturated saline once, dried with anhydrous sodium sulfate, concentrated, and passed through the column (PE: EA=20:1) to obtain product, 497mg, yield 87%.

### 4) Preparation of compound B34

497 mg of compound B34 (0.8 mmol) was dissolved in 10 ml of dichloromethane and 2 ml of trifluoroacetic acid was added dropwise under an ice bath and the reaction was carried out at room temperature for 3h. TLC monitoring showed the reaction was complete, the mixture was concentrated, diluted by adding 60 ml of ethyl acetate and washed three times with water, washed with saturated saline three times, dried with anhydrous sodium sulfate, concentrated, and passed through the column (DCM: MeOH =10:1) to obtain product, 422mg, yield 90%.

### 5) Preparation of compound B36

54mg B34(0.1mmol), 20mg B35(0.12mmol), 58mg HATU(0.15mmol) were dissolved in 5ml dry DMF, nitrogen gas was exchanged, 0.2ml DIPEA was added, and the reaction was carried out at 60°C for 4h. TLC monitoring showed the reaction was complete, the mixture was cooled to room temperature, diluted with 60 ml of water, extracted with EA three times. The organic phases were combined, washed with saturated saline once, dried with anhydrous sodium sulfate, concentrated, and passed through the column (PE: EA=5:1) to obtain product, 51 mg, yield 77%.

### 6) Preparation of compound M36

51 mg of compound M36 (0.07 mmol) was dissolved in 10 ml of methanol, 1 ml of water, 51 mg of NaOH was added, the reaction was carried out at 60 °C overnight. TLC monitoring showed the reaction was complete, the methanol was spun off, the residue was diluted with 30 ml of water, adjusted to acidity by 1 M HCl, and extracted three times with ethyl acetate. The organic phases were combined, washed with saturated saline, dried with anhydrous sodium sulfate, concentrated, and passed through the column to obtain product, 23mg, colorless oil, yield 47%. 1H NMR (400 MHz, CDCl3) δ 10.27 (s, 2H), 8.19 (d, J = 3.0 Hz, 1H), 7.51 (dd, J = 11.3, 4.3 Hz, 1H), 7.37 (s, 4H), 6.66 (d, J = 7.6 Hz, 2H), 5.67 (t, J = 7.0 Hz, 1H), 3.73 (d, J = 122.7 Hz, 8H), 2.48 (s, 2H), 2.17 (d, J = 9.0 Hz, 2H), 1.47 (d, J = 35.6 Hz, 6H), 1.21 (dd, J = 47.1, 14.9 Hz, 26H).

Using B7 and B26 as raw materials and exchanging different substrates for B35, the following compounds can be synthesized using a route similar to the one described above.

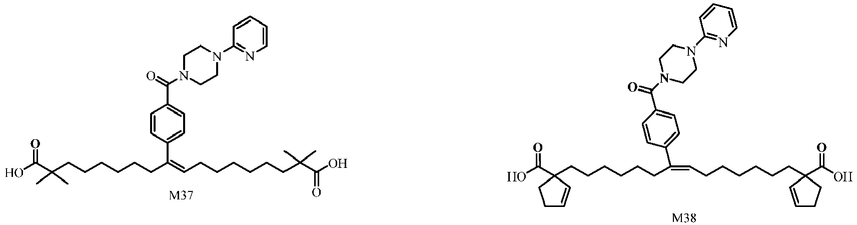

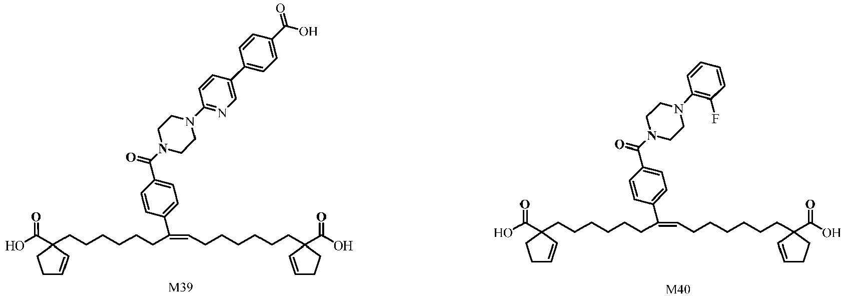

Compound M37: 1H NMR (400 MHz, CDCl3) δ 8.20 (d, J = 2.7 Hz, 1H), 7.52 (t, J = 7.3 Hz, 1H), 7.41 (d, J = 7.4 Hz, 2H), 7.16 (d, J = 7.5 Hz, 2H), 6.68 (d, J = 8.4 Hz, 2H), 5.43 (d, J = 6.9 Hz, 1H), 3.75 (d, J = 122.3 Hz, 8H), 2.31 (s, 2H), 1.88 (d, J = 6.1 Hz, 2H), 1.46 (s, 4H), 1.35 - 1.10 (m, 28H).

Compound M38: 1H NMR (400 MHz, CDCl3) δ 8.20 (d, J = 3.8 Hz, 1H), 7.56 - 7.48 (m, 1H), 7.42 - 7.32 (m, 4H), 6.67 (t, J = 6.9 Hz, 2H), 5.69 (dd, J = 18.2, 10.7 Hz, 5H), 3.64 (t, J = 81.7 Hz, 9H), 2.29 (dd, J = 91.6, 33.5 Hz, 11H), 1.82 - 1.07 (m, 25H).

Compound M39: 1H NMR (400 MHz, CDCl3) δ 8.44 (s, 1H), 8.01 (d, J = 8.1 Hz, 2H), 7.90 (d, J = 6.8 Hz, 1H), 7.58 (d, J = 8.0 Hz, 2H), 7.44 (d, J = 3.1 Hz, 4H), 6.95 (d, J = 8.9 Hz, 1H), 5.71 (s, 5H), 3.76 (d, J = 93.9 Hz, 8H), 2.54 (s, 2H), 2.34 (s, 6H), 2.22 (d, J = 7.2 Hz, 2H), 1.73 (d, J = 12.1 Hz, 6H), 1.47 (s, 2H), 1.28 (d, J = 25.8 Hz, 14H).

Compound M40: 1H NMR (400 MHz, CDCl3) δ 7.37 (s, 4H), 7.13 - 7.01 (m, 2H), 7.01 - 6.90 (m, 2H), 5.85 - 5.63 (m, 5H), 3.69 (s, 4H), 3.06 (s, 5H), 2.49 (s, 2H), 2.33 (s, 6H), 2.18 (s, 2H), 1.78 - 1.48 (m, 7H), 1.43 (s, 2H), 1.32 - 1.17 (m, 14H).

### Example 7 Biological experimental tests

### 7.1 ACLY enzyme activity test

### Instrument: Envision (PerkinElmer, USA)

Materials: Acl---- human ACLY / acly / ATP citrate lyase protein (His tag) was purchased from Sino Biological; kinase assay kit ADP-GLO (promega #V9102).

Experimental principle: In this experiment, the ATP-dependent citrate lyase ACL catalyzes the conversion of citrate to acetyl coenzyme A, which in turn produces malonyl coenzyme A, a precursor molecule for fatty acid synthesis, and this reaction is accompanied by ATP depletion, so the change in ATP can be detected using the ADP-Glo Kinase Assay (#V9102) to indirectly reflect the inhibitory effect of the compound on the enzymatic activity of ACL. This kinase detection kit can quantitatively determine the amount of ADP catalyzed by ACLY enzyme activity. (First, the compound, the enzyme and the substrate are incubated at 37 degrees for half an hour, ADP-Glo&#V9102; reagent is added and incubated for half an hour to terminate the reaction and consume the remaining ATP; then the kinase detection reagent is added (which converts the ADP to ATP while also detecting the newly synthesized ATP using the coupled fluorophore enzyme / fluorescein reaction) and incubated for half an hour. The value is read by using Envision.

Experimental method: ADP Glo luminescence method was used for determination. It reflects the activity of ACLY enzyme by quantitatively detecting the amount of ADP, and the enzymatic reaction catalyzed by ACLY is proportional to the amount of ADP detected by the luminescent signal. Firstly, the compound is diluted with 10% DMSO, and then 1µl of compound dilution is added to a 5µl reaction system, so that the final reaction system has a DMSO content of 2%. The enzymatic reaction catalyzed by ACLY was carried out at 37 °C for 30 minutes. 5 µl reaction mixture contained the following components: 40 mM Tris, pH 8.0, 10 mM MgCl₂, 5 mM DTT, ATP, CoA, sodium citrate, and ACLY. After the enzymatic reaction was complete, 2.5µl of ADP Glo reagent was added to each reaction system and incubated at room temperature for 1 hour. Afterwards, 5 µl of kinase detection reagent was added and incubated at room temperature for 30 minutes. The luminous signal was detected using Envision (PerkinElmer, USA).

Data processing: For compound, the activity dose dependent relationship was tested. IC50 value was obtained by non-linear fitting of sample concentration based on sample activity. The software used for calculation was Graphpad Prism 7, and the model used for fitting was sigmoid dose response (variable slope). For most inhibitor screening models, the bottom and top of the fitting curve were set to 0 and 100. In general, each sample is set with multiple wells (n ≥ 2) during testing, which are represented in the results as Standard Deviation (SD) or Standard Error (SE).

The experimental results are shown in Table 1 below. NDI-091143 is a reported ACLY inhibitor used as a positive control.

**Table 1 Inhibitory activity of example compounds on ACLY**

| **No.** | **IC50 (µM)** | **No.** | **IC50 (µM)** |
|---|---|---|---|
| | **Human ACLY** | | **Human ACLY** |
| M1 | 1.7±0.3 | M22 | 4.9±3.3 |
| M2 | 2.9±0.95 | M23 | 3.3±0.72 |
| M3 | 1.0±0.13 | M24 | 5.5±1.9 |
| M4 | 22.6±2.7 | M25 | 4.0±0.86 |
| M5 | 1.5±0.45 | M26 | 9.4±5.4 |
| M6 | 5.3±0.98 | M27 | 7.7±0.39 |
| M7 | 4.1±1.7 | M28 | 17.0±2.5 |
| M8 | 1.8±0.39 | M29 | 5.8±1.8 |
| M9 | 11.2±1.61 | M30 | 19.9±7.8 |
| M10 | 1.9±0.2 | M31 | 8.5±3.2 |
| M11 | 1.9±0.31 | M32 | 4.6±1.3 |
| M12 | 3.9±0.2 | M33 | 3.3±0.45 |
| M13 | 2.7±0.8 | M34 | 10.2±0.3 |
| M14 | 7.0±0.16 | M35 | 0.9±0.1 |
| M15 | 52.2±0.27 | M36 | 4.57±0.41 |
| M16 | 4.0±1.31 | M37 | 5.21±0.27 |
| M17 | 3.1±0.52 | M38 | 0.76±0.01 |
| M18 | 16.5±2.21 | M39 | 1.30±0.15 |
| M19 | 3.1±1.3 | M40 | 1.69±0.2 |
| M20 | 3.5±1.7 | DN1-091143 | 0.0021±0.0004 |
| M21 | 3.8±0.83 | | |

NDI-091143, a reported ACLY inhibitor, was used as a positive control with the following structure:

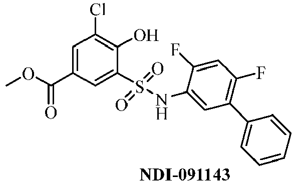

### 7.2 MTS experiment

### Experimental instrument: Molecular Device VersaMax

Experimental materials: NCI-H358 cells (H358, human non-small cell lung cancer cells) were purchased from the cell bank/stem cell bank of the Chinese Academy of Sciences; RPMI-1640 culture medium (GIBCO, product number 3180002), and human tumor cell non-small cell lung cancer cell A549 were gifted by the research group of researcher Li Jia from SHANGHAI INSTITUTE OF MATERIA MEDICA, CHINESE ACADEMY OF SCIENCES. CellTier 96 ^{®} The Aquarius One Solution Cell Promotion Assay (MTS) was purchased from Promega # G3581.

Experimental principle: CellTier 96 ^{®} AQueous One Solution Cell Proliferation Assay (MTS) colorimetric method was used to detect cell proliferation. This analysis method is based on the tetrazole compound (3-(4,5-dimethylthiazol-2-yl)-5-(3-carboxymethoxyphenyl)-2-(4-sulfophenyl)-2H-tetrazolium, internal salt; MTS). The conversion of yellow MTS to blue purple water-soluble formazan is catalyzed by dehydrogenase, which exists in living cells with metabolic activity; Dead cells cannot complete the transformation from MTS to formamide. The amount of formazan determined by absorbance at 490nm is directly proportional to the number of live cells in culture.

### Experimental method:

Sample processing: Dissolve the sample in DMSO and store it at low temperature. The concentration of DMSO in the final system should be controlled within the range that does not affect the detection activity.

Cell survival rate was detected by using MTS method, that is, adherent cells growing in logarithmic phase were digested with 0.025% trypsin, and counted; 80 µl cells were inoculated into a 96 well plate at a density of 3000 cells/well for A549 and 5000 cells/well for H358 (all cultured under 1% FBS conditions), and grown overnight in a 5% CO₂ and 37 °C incubator. Each compound was diluted with a three-fold concentration gradient, eight concentrations were tested, and three wells were set for each concentration. 20 µl of the diluted compounds was added to the corresponding cell wells. Adherent cells A549 and H358 were cultured in a 5% CO₂ and 37 °C incubator for 72 hours; 20 µl of MTS was added separately and incubated for 2 hours in a 5% CO₂ and 37 °C incubator. Molecular Device VersaMax was used to test the light absorption value at 490nm (L1), with a reference wavelength of 690nm (L2). The (L1-L2) values were plotted against different concentrations of inhibitors and IC₅₀ was obtained by fitting the formula.

### Data Processing:

The experimental results were fitted to the IC₅₀ using Graphpad Prism 7. The model used for fitting was log(inhibitor) vs Response---variable slop, and the results of each assay were equipped with triple replicate wells to ensure the accuracy of the experiment. The results were expressed as Standard Deviation (SD) or Standard Error (SE).

Similarly, the antiproliferative assays of the compounds on pancreatic cancer cell line ASPC-1, human pancreatic cancer cell MIA-PACA and breast cancer cell MCF-7 were performed as above.

### Results:

The inhibitory activities of compounds M5, M19, M25 and M29 on the proliferation of each cancer cell are shown in Table 2 below, where 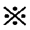 denotes that it has not been tested and NDI-091143, a reported ACLY inhibitor, was used as a positive control.

**Table 2 Inhibitory activity of representative compounds on the proliferation of each cancer cell in vitro**

| **Cell line** | **IC₅₀ (µM)** | | | | |
|---|---|---|---|---|---|
| | **M5** | **M19** | **M25** | **M29** | **NDI-091143** |
| **A549** | 12.5±1.2 | 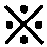 | 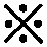 | 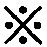 | 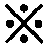 |
| **H358** | 23.6±1.9 | 3.5±0.4 | 5.6±0.5 | 9.0±1.9 | 50.2±27.7 |
| **MCF-7** | 31.8±1.7 | 3.9±0.5 | 5.7±1.5 | 6.5±0.4 | 84.8±45.3 |
| **ASPC-1** | 27.3±0.8 | 2.7±1.0 | 29.2±11.4 | 28.0±10.1 | 83.3±34.7 |
| **MIA-PACA** | 13.5±18 | 1.9±0.5 | 11.9±2.7 | 12.8±1.6 | 40.9±14.3 |

The compounds described herein have inhibitory activity against ACLY with cellular activity superior to that of NDI-091143. This is due to the fact that the compounds of the present invention have hydrophobic long chains with better membrane permeability than the benzenesulfonamide inhibitor NDI-091143. The compounds described herein have excellent drug-like properties and have more potential to be inhibitors targeting ACLY for the preparation of drugs applied to metabolic disease or cancer.

All literature referred to in the present invention is cited as a reference in this application as if each literature is cited individually as a reference. It is further to be understood that after reading the foregoing teachings of the present invention, a person skilled in the art may make various alterations or modifications to the present invention, which equivalent forms likewise fall within the scope of the claims appended to the present application.

## Claims

1. A compound represented by general formula (I), or a stereoisomer, enantiomer, diastereomer, racemate or pharmaceutically acceptable salt thereof, 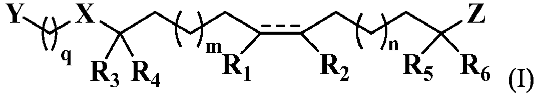
wherein --- represents a double bond or a single bond;
R₁ and R₂ are each independently H, C1-C4 alkyl, C2-C4 alkenyl, C2-C4 alkynyl, C3-C7 cycloalkyl, C3-C7 cycloalkenyl or C6-C10 aryl; or, R₁ and R₂ together with the attached carbons form a saturated 3-7 membered ring, or a 3-7 membered ring containing an unsaturated double bond;
R₃ and R₄ are each independently H, C1-C4 alkyl, C2-C4 alkenyl, C2-C4 alkynyl, C3-C7 cycloalkyl, C3-C7 cycloalkenyl or C6-C10 aryl; or, R₃ and R₄ together with the attached carbon form a saturated 3-7 membered ring, or a 3-7 membered ring containing an unsaturated double bond;
R₅ and R₆ are each independently H, C1-C4 alkyl, C2-C4 alkenyl, C2-C4 alkynyl, C3-C7 cycloalkyl, C3-C7 cycloalkenyl or C6-C10 aryl; or, R₅ and R₆ together with the attached carbon form a saturated 3-7 membered ring, or a 3-7 membered ring containing an unsaturated double bond;
m, n and q are each independently 0, 1, 2, 3, 4, 5 or 6;
Z is -OH, -COOH, -COOR₇, -SO₃H or -CONHR₇, wherein R₇ is C1-C4 alkyl;
X is -CO-, -O-, -NH-, -S-, -CH₂-, 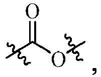 -CONH-, 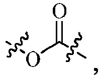 -NHOC-, 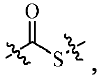 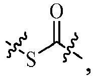 -CH₂CO- or -COCH₂-;
Y is H, C6-C10 aryl, C1-C4 alkyl or adamantyl (Ad-); or Y is: 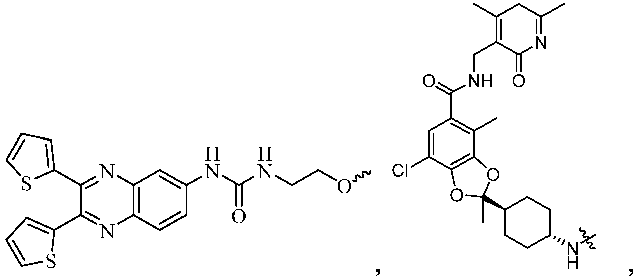 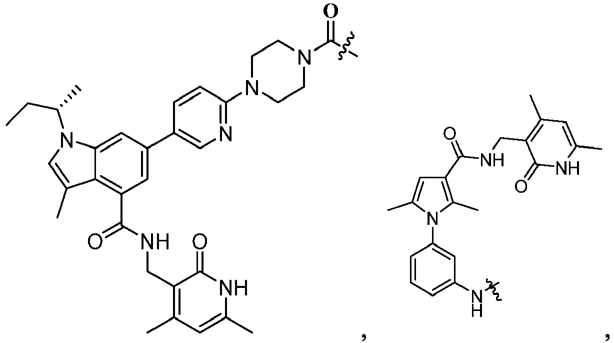 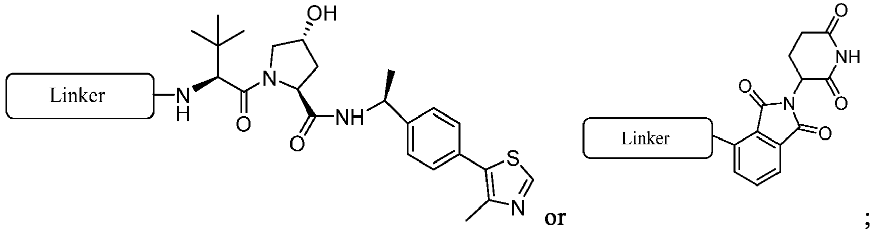
wherein the linker is a C1-C20 alkylene or polyethylene glycol, or there is no such linker;
each of the above-mentioned C1-C4 alkyl, C2-C4 alkenyl, C2-C4 alkynyl, C3-C7 cycloalkyl, C3-C7 cycloalkenyl or C6-C10 aryl is unsubstituted or substituted, wherein the "substituted" means a substitution with 1, 2, 3, 4 or 5 substituents selected from the group consisting of a halogen, C1-C4 alkyl, C1-C4 alkoxy, C3-C7 cycloalkyl, C3-C7 cycloalkenyl and C6-C10 aryl.

2. The compound according to claim 1, or the stereoisomer, enantiomer, diastereomer, racemate or pharmaceutically acceptable salt thereof, wherein the compound has a structure shown in formula II:

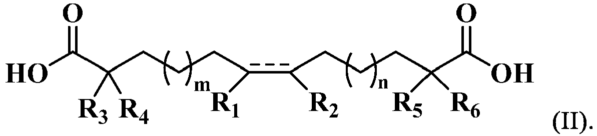

3. The compound according to claim 1, or the stereoisomer, enantiomer, diastereomer, racemate or pharmaceutically acceptable salt thereof, wherein the compound has a structure shown in formula III:

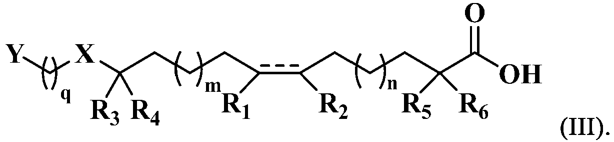

4. The compound according to claim 1, or the stereoisomer, enantiomer, diastereomer, racemate or pharmaceutically acceptable salt thereof, wherein the compound has a structure shown in formula IV: 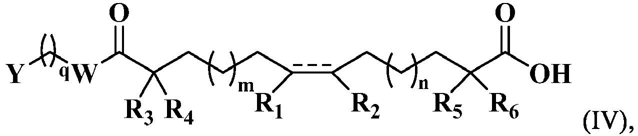 W is absent, -O-, -NH-, -S- or -CH2-.

5. The compound according to claim 1, or the stereoisomer, enantiomer, diastereomer, racemate or pharmaceutically acceptable salt thereof, wherein R₁ and R₂ are each independently H, methyl, ethyl, propyl, vinyl, propenyl, ethynyl, propynyl, C3-C6 cycloalkyl, C3-C6 cycloalkenyl or C6-C10 aryl; or, R₁ and R₂ together with the attached carbons form a saturated 3-6 membered ring, or a 3-6 membered ring containing one unsaturated double bond;
R₃ and R₄ are each independently H, methyl, ethyl, propyl, vinyl, propenyl, ethynyl, propynyl, C3-C6 cycloalkyl, C3-C6 cycloalkenyl or C6-C10 aryl; or, R₃ and R₄ together with the attached carbon form a saturated 3-6 membered ring, or a 3-6 membered ring containing one unsaturated double bond;
R₅ and R₆ are each independently H, methyl, ethyl, propyl, vinyl, propenyl, ethynyl, propynyl, C3-C6 cycloalkyl, C3-C6 cycloalkenyl or C6-C10 aryl; or, R₅ and R₆ together with the attached carbon form a saturated 3-6 membered ring, or a 3-6 membered ring containing one unsaturated double bond.

6. The compound according to claim 1, or the stereoisomer, enantiomer, diastereomer, racemate or pharmaceutically acceptable salt thereof, wherein m and n are each independently 1, 2, 3, 4 or 5; and/or q is 0, 1, 2, 3 or 4.

7. The compound according to claim 1, or the stereoisomer, enantiomer, diastereomer, racemate or pharmaceutically acceptable salt thereof, wherein R₁ and R₂ are each independently H, C1-C4 alkyl, C2-C4 alkenyl, C2-C4 alkynyl, C3-C7 cycloalkyl, C3-C7 cycloalkenyl or C6-C10 aryl;
the above-mentioned C1-C4 alkyl, C2-C4 alkenyl, C2-C4 alkynyl, C3-C7 cycloalkyl, C3-C7 cycloalkenyl or C6-C10 aryl is unsubstituted or substituted, wherein the "substituted" means a substitution with 1, 2, 3, 4 or 5 substituents selected from the group consisting of: a halogen, C1-C4 alkyl, C1-C4 alkoxy, C3-C7 cycloalkyl, C3-C7 cycloalkenyl, C6-C10 aryl, and -CO-(5-7 membered heterocyclyl)-(5-7 membered heteroaryl); the above group is optionally further substituted with a substituent selected from the following group: a C6-C10 aryl, halogenated C6-C10 aryl, carboxyl substituted C6-C10 aryl, C1-C4 alkyl substituted C6-C10 aryl, C1-C4 haloalkyl substituted C6-C10 aryl.

8. The compound according to claim 1, or the stereoisomer, enantiomer, diastereomer, racemate or pharmaceutically acceptable salt thereof, wherein the compound is selected from the following group:

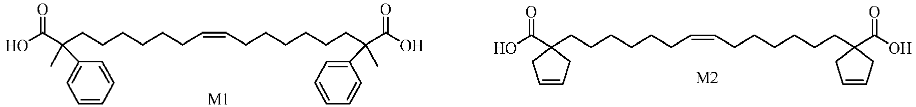

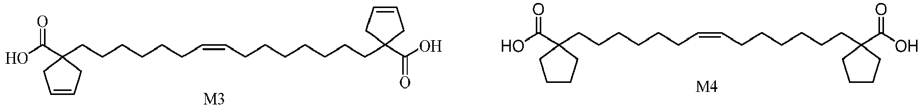

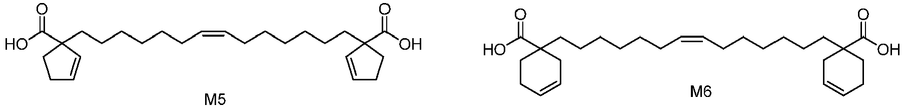

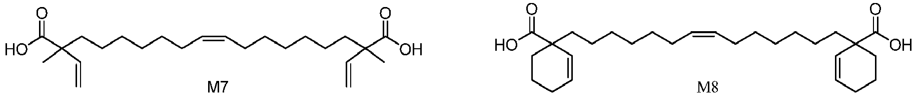

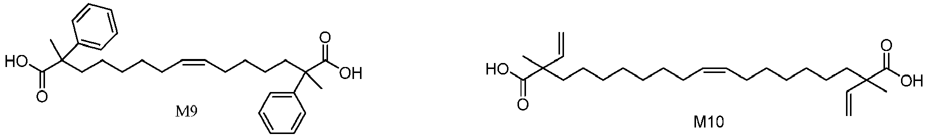

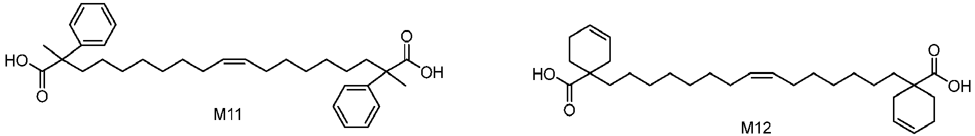

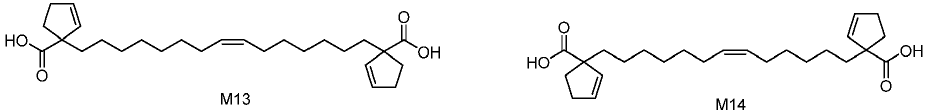

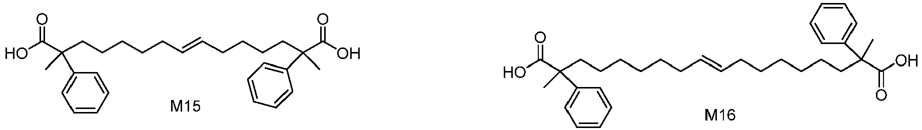

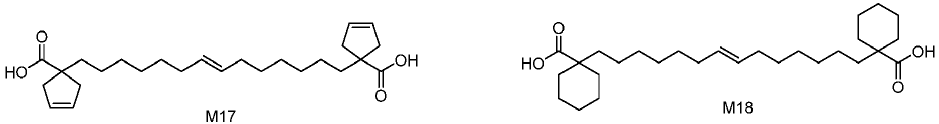

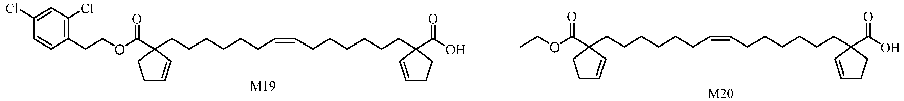

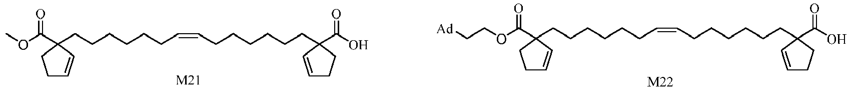

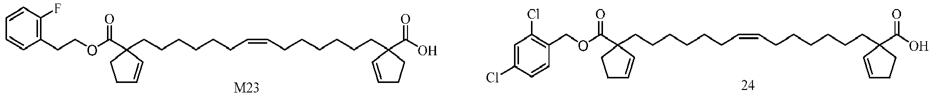

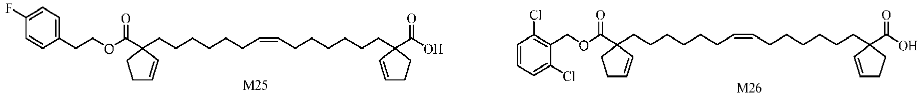

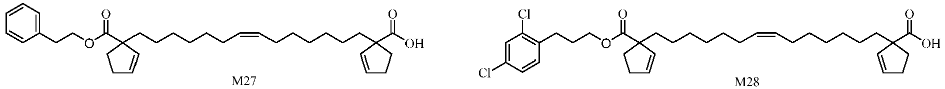

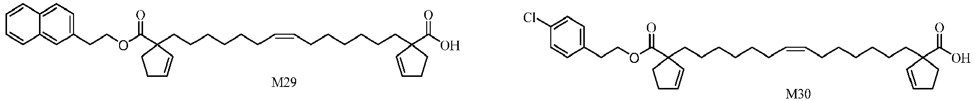

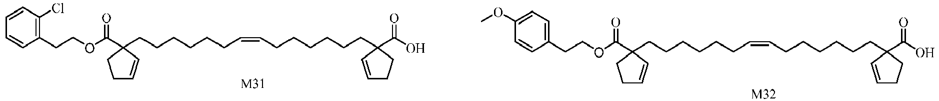

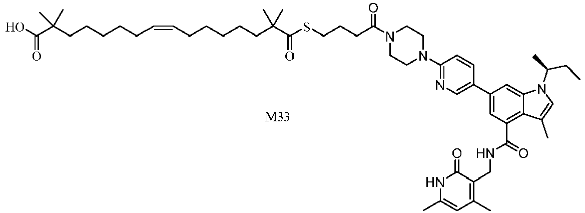

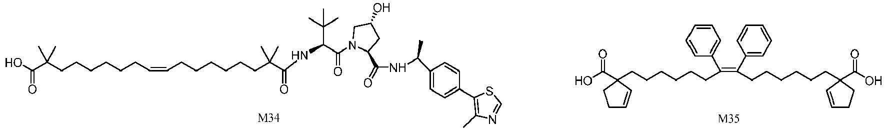

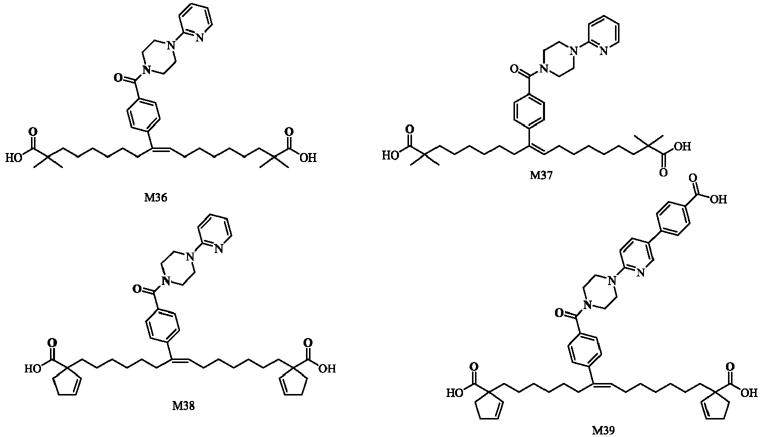

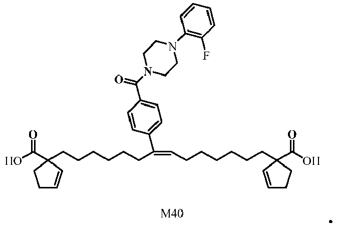

9. A pharmaceutical composition, comprising the compound represented by the general formula (I) according to any one of claims 1 to 8, or the stereoisomer, enantiomer, diastereomer, racemate or pharmaceutically acceptable salt thereof; and a pharmaceutically acceptable carrier.

10. Use of the compound according to any one of claims 1 to 8, or the stereoisomer, enantiomer, diastereomer, racemate or pharmaceutically acceptable salt thereof or the pharmaceutical composition according to claim 9 for preparing ACLY inhibitors; or for preparing a medicament for preventing and/or treating a metabolic disease or cancer.

11. The use according to claim 10, wherein the metabolic disease is selected from the group consisting of hyperlipidemia, atherosclerosis, non-alcoholic fatty liver disease, and diabetes;
the cancer is selected from the group consisting of lung cancer, pancreatic cancer, breast cancer, ovarian cancer, liver cancer, bowel cancer, brain cancer, and acute myeloid leukemia.
